(19) Europäisches Patentamt
European Patent Office
Office européen des brevets

(11) EP 1 599 878 B1

(12) # EUROPEAN PATENT SPECIFICATION

(45) Date of publication and mention of the grant of the patent:
**07.06.2006 Bulletin 2006/23**

(21) Application number: **04713552.0**

(22) Date of filing: **23.02.2004**

(51) Int Cl.:
***G11B 7/24*** (2006.01) ***C07D 403/02*** (2006.01)

(86) International application number:
**PCT/EP2004/050185**

(87) International publication number:
**WO 2004/079732 (16.09.2004 Gazette 2004/38)**

(54) **OPTICAL RECORDING MATERIALS HAVING HIGH STORAGE DENSITY**
MATERIALIEN FÜR OPTISCHE AUFZEICHNUNG MIT HOHER SPEICHERDICHTE
MATERIAUX D'ENREGISTREMENT OPTIQUE A DENSITE DE MEMORISATION ELEVEE

(84) Designated Contracting States:
**AT BE BG CH CY CZ DE DK EE ES FI FR GB GR HU IE IT LI LU MC NL PT RO SE SI SK TR**

(30) Priority: **03.03.2003 CH 3312003**

(43) Date of publication of application:
**30.11.2005 Bulletin 2005/48**

(73) Proprietor: **Ciba Specialty Chemicals Holding Inc.
4057 Basel (CH)**

(72) Inventors:
- **LEHMANN, Urs
CH-4057 Basel (CH)**
- **SUTTER, Peter
CH-4132 Muttenz (CH)**
- **SCHMIDHALTER, Beat
CH-4416 Bubendorf (CH)**
- **BUDRY, Jean-Luc
CH-2842 Rossemaison (CH)**

(56) References cited:
**EP-A- 1 130 584**
**DE-A- 1 932 894**
**US-A- 5 962 657**
**US-B1- 6 376 664**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 599 878 B1

## Description

The invention relates to new optical recording materials that have excellent recording and playback quality especially at a wavelength of 350-500 nm. Recording and playback can be effected very advantageously with high sensitivity at the same wavelength, and the storage density that is achievable is significantly higher than in the case of known materials. In addition, the materials according to the invention have very good storage properties before and after recording, even under especially harsh conditions, such as exposure to sunlight or fluorescent lighting, heat and/or high humidity. In addition, their manufacture is simple and readily reproducible using customary coating processes, such as spin-coating.

WO 02/082438 discloses the use of ionic salts, including those with metal complex anions, for optical recording materials. Those colorants are always substituted by alkyl, alkenyl, aryl or heteroaryl at the nitrogen atom. Their optical properties do not, however, fully satisfy increased demands. In particular, the refractive index as well as the absorption and the steepness of the absorption band on its long wavelength flank in the solid still leave something to be desired.

JP-A-11/34500, JP-A-19/92479 and EP-A-0 903 733 disclose metal and boron complexes of colorants of formulae

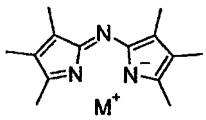

and

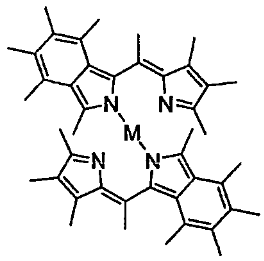

, '

which can be used at from 520 to 690 nm for optical recording materials such as CD-R or DVD-R. Here too, however, the optical properties, especially the spectral properties in or near the UV range that are necessary for the highest possible storage densities, and the information density per unit surface area are not able to satisfy the highest demands as desired. The information density per unit surface area is far lower than is desirable.

Conventional optical recording materials therefore satisfy high demands only to some extent, or do not satisfy all demands to an entirely satisfactory degree at the same time.

On the other hand, J. Org. Chem. 67/16, 5753-5772 [2002] describes the synthesis of a number of bis(*o*-azaheteroaryl)methanes and their coordination properties with respect to divalent transition metals, heteroaryl being 1,3-azol-2-yl, 1,3-benzazol-2-yl and azinyl and the transition metals being Zn, Cu, Co, Ni, Hg and Pd. *Inter alia* 2:1 salt complexes of bis(benzothiazol-2-yl)methane and bis(benzoxazol-2-yl)methane with copper(II) chloride and nickel(II) sulfate, and cobalt(II) chloride and palladium(II) nitrate are disclosed, whereas bis(thiazol-2-yl)methane yields, with deprotonation, neutral 2:1 chelates with Zn(II), Cu(II), Ni(II) and Co(II). All substances are strongly coloured.

The aim of the invention is an optical recording medium having high information density, sensitivity and data reliability. Such a recording medium should be robust, durable and easy to use. Furthermore, it should be inexpensive to manufacture as a mass-produced product and should require equipment that is as small and inexpensive as possible.

The invention therefore relates to an optical recording medium comprising a substrate, a recording layer and optionally one or more reflecting layers, wherein the recording layer comprises a compound of formula

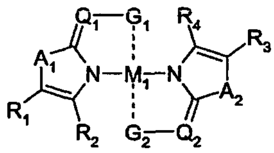 (I)

or a tautomer thereof, wherein $G_1$ and $G_2$ are each independently of the other

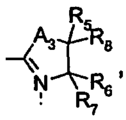, 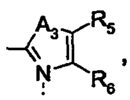, 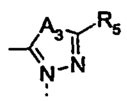 or 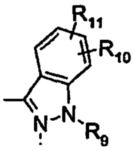,

$A_1$ and $A_2$ are each independently of the other $N(R_{12})$, O, S or Se and $A_3$ is $C(C_1\text{-}C_5alkyl)_2$, $C(C_4\text{-}C_5alkylene)$, $N(R_{12})$, O, S, Se, $N{=}C(R_{13})$ or unsubstituted or $R_{14}$-substituted CH=CH;

$M_1$ is a transition metal of groups IX to XII, preferably Co, Cu, Ni, Pd or Zn, especially Co, Cu or Ni;

$Q_1$ and $Q_2$ are each independently of the other $C(R_{15})$, N or P;

$R_1$, $R_2$, $R_3$, $R_4$, $R_5$, $R_6$, $R_7$, $R_8$ and $R_{14}$ are each independently of the others hydrogen, $R_{18}$, or $C_6$-$C_{12}$aryl, $C_4$-$C_{12}$heteroaryl, $C_7$-$C_{12}$aralkyl or $C_5$-$C_{12}$heteroaralkyl each unsubstituted or substituted by one or more, where applicable identical or different, radicals $R_{18}$; or

$R_1$ and $R_2$, $R_3$ and $R_4$, $R_5$ and $R_6$, $R_5$ and $R_{13}$ and/or $R_5$ and $R_{14}$, together in pairs, are $C_3$-$C_6$alkylene or $C_3$-$C_6$alkenylene, each of which is unsubstituted or substituted by one or more, where applicable identical or different, radicals $R_{17}$ and may be uninterrupted or interrupted by O, S or $N(R_{12})$, or 1,4-buta-1,3-dienylene,

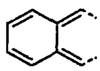

or

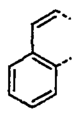,

each of which is unsubstituted or substituted by one or more, where applicable identical or different, radicals $R_{18}$ and in which 1 or 2 carbon atoms may have been replaced by nitrogen;

$R_9$, $R_{12}$ and $R_{13}$ are each independently of the others $C_1$-$C_{24}$alkyl, $C_3$-$C_{24}$cyclalkyl, $C_2$-$C_{24}$alkenyl, $C_3$-$C_{24}$cycloalkenyl, $C_1$-$C_4$alkyl-$[O\text{-}C_1\text{-}C_4alkylene]_m$ or $C_1$-$C_4$alkyl-$[NH\text{-}C_1\text{-}C_4alkylene]_m$, each of which is unsubstituted or substituted by one or more, where applicable identical or different, radicals $R_{17}$; or $C_6$-$C_{12}$aryl, $C_4$-$C_{12}$heteroaryl, $C_7$-$C_{12}$aralkyl or $C_5$-$C_{12}$heteroaralkyl, each of which is unsubstituted or substituted by one or more, where applicable identical or different, radicals $R_{18}$;

$R_{10}$, $R_{11}$ and $R_{18}$ are each independently of the others halogen, nitro, cyano, thiocyanato, hydroxy, O-$R_{19}$, O-CO-$R_{19}$, S-$R_{19}$, CHO, $COR_{20}$, $CHOR_{19}OR_{23}$, $CR_{20}OR_{19}OR_{23}$, $R_{16}$, N=N-$R_{16}$, $N{=}CR_{19}R_{20}$, $N{=}CR_{21}R_{22}$, $C(R_{15}){=}NR_{19}$, $C(R_{15}){=}NR_{21}$ , $C(R_{15}){=}CR_{21}R_{22}$, $NH_2$, NH-$R_{19}$, $NR_{19}R_{20}$, $NH_3^+$, $NH_2R_{19}^+$, $NHR_{19}R_{20}^+$, $NR_{19}R_{20}R_{23}^+$, $CONH_2$, $CONHR_{19}$, $CONR_{19}R_{20}$. $SO_2R_{19}$, $SO_2NH_2$, $SO_2NHR_{19}$, $SO_2NR_{19}R_{20}$, COOH, $COOR_{19}$, $OCOOR_{19}$, $NHCOR_{19}$, $NR_{19}COR_{23}$, $NHCOOR_{19}$, $NR_{19}COOR_{23}$, ureido, $NR_{19}$-CO-$NHR_{23}$, $B(OH)_2$, $B(OH)(OR_{19})$, $B(OR_{19})OR_{23}$, phosphato, $PR_{19}R_{23}$, $POR_{19}OR_{23}$, $P({=}O)OR_{19}OR_{23}$, $OPR_{19}R_{23}$, $OPR_{19}OR_{23}$, $OP({=}O)R_{19}OR_{23}$, $OP({=}O)OR_{19}OR_{23}$, $OPO_3R_{19}$, sulfato, sulfo, or $C_1$-$C_{12}$alkyl, $C_3$-$C_{12}$cycloalkyl, $C_1$-$C_{12}$alkylthio, $C_3$-$C_{12}$cycloalkylthio, $C_1$-$C_{12}$alkoxy or $C_3$-$C_{12}$cycloalkoxy each unsubstituted or substituted by one or more, where applicable identical or different, radicals $R_{17}$;

$R_{15}$ is hydrogen, cyano, hydroxy, $C_1$-$C_{12}$alkoxy, $C_3$-$C_{12}$cycloalkoxy, $C_1$-$C_{12}$alkylthio, $C_3$-$C_{12}$cycloalkylthio, amino, $NHR_{24}$, $NR_{25}R_{26}$, $R_{27}$, halogen, nitro, formyl, N=N-$R_{27}$, $C(R_{14}){=}CR_{21}R_{22}$, $C(R_{14}){=}NR_{19}$, COO-$R_{25}$, carboxy, carbamoyl,

$CONH-R_{25}$, $CONR_{25}R_{26}$, $N=CR_{19}R_{20}$, or $C_1$-$C_{12}$alkyl, $C_3$-$C_{12}$cycloalkyl, $C_2$-$C_{12}$alkenyl or $C_3$-$C_{12}$cycloalkenyl each unsubstituted or substituted by one or more, where applicable identical or different, halogen, hydroxy, $C_1$-$C_{12}$alkoxy or $C_3$-$C_{12}$cycloalkoxy radicals;

$R_{16}$ is $C_6$-$C_{12}$aryl, $C_4$-$C_{12}$heteroaryl, $C_7$-$C_{12}$aralkyl or $C_5$-$C_{12}$heteroaralkyl, each of which is unsubstituted or substituted by one or more, where applicable identical or different, radicals $R_{28}$;

$R_{17}$ is halogen, hydroxy, $O-R_{25}$, $O-CO-R_{25}$, $S-R_{25}$, $NH_2$, $NH-R_{25}$, $NR_{25}R_{26}$, $NH_3^+$, $NH_2R_{25}^+$, $NHR_{25}R_{26}^+$, $NR_{24}R^{25}R_{26}^+$, $NR_{25}-CO-R_{24}$, $NR_{25}COOR_{24}$, cyano, formyl, $COO-R_{25}$, carboxy, carbamoyl, $CONH-R_{25}$, $CONR_{25}R_{26}$, ureido, $NH-CO-NHR_{24}$, $NR_{25}-CO-NHR_{24}$, phosphato, $PR_{25}R_{24}$, $POR_{25}OR_{24}$, $P(=O)OR_{25}OR_{24}$, $OPR_{25}R_{24}$, $OPR_{25}OR_{24}$, $OP(=O)R_{25}OR_{24}$, $OPO_3R_{25}$, $OP(=O)OR_{25}OR_{24}$, $SO_2R_{25}$, sulfato, sulfo, $R_{27}$, $N=N-R_{27}$, or $C_1$-$C_{12}$alkoxy or $C_1$-$C_{12}$cycloalkoxy each unsubstituted or mono- or poly-substituted by halogen;

$R_{19}$, $R_{20}$ and $R_{23}$ are each independently of the others $R_{16}$, or $C_1$-$C_{12}$alkyl, $C_3$-$C_{12}$cycloalkyl, $C_2$-$C_{12}$alkenyl or $C_3$-$C_{12}$cycloalkenyl each unsubstituted or substituted by one or more, where applicable identical or different, halogen, hydroxy, $C_1$-$C_{12}$alkoxy or $C_3$-$C_{12}$cycloalkoxy radicals; or

$R_{14}$ and $R_{19}$ together, $R_{15}$ and $R_{19}$ together and/or $R_{19}$ and $R_{23}$ together are $C_2$-$C_{12}$alkylene, $C_3$-$C_{12}$cycloalkylene, $C_2$-$C_{12}$alkenylene or $C_3$-$C_{12}$cycloalkenylene, each of which is unsubstituted or substituted by one or more, where applicable identical or different, halogen, hydroxy, $C_1$-$C_{12}$alkoxy or $C_3$-$C_{12}$cycloalkoxy radicals; or

$R_{19}$ and $R_{20}$ together with the common nitrogen are pyrrolidine, piperidine, piperazine or morpholine, each of which is unsubstituted or mono- to tetra-substituted by $C_1$-$C_4$alkyl; or carbazole, phenoxazine or phenothiazine, each of which is unsubstituted or substituted by one or more, where applicable identical or different, radicals $R_{28}$;

$R_{21}$ and $R_{22}$ are each independently of the other $NR_{25}R_{26}$, CN, $CONH_2$, $CaNHR_{19}$, $CONR_{19}R_{20}$ or $COOR_{20}$;

$R_{24}$, $R_{25}$ and $R_{26}$ are each independently of the others $C_1$-$C_{12}$alkyl, $C_3$-$C_{12}$cycloalkyl, $C_2$-$C_{12}$alkenyl, $C_3$-$C_{12}$cycloalkenyl, $C_6$-$C_{12}$aryl, $C_4$-$C_{12}$heteroaryl, $C_7$-$C_{12}$aralkyl or $C_5$-$C_{12}$heteroaralkyl; or

$R_{25}$ and $R_{26}$ together with the common nitrogen are pyrrolidine, piperidine, piperazine or morpholine, each of which is unsubstituted or mono- to tetra-substituted by $C_1$-$C_4$alkyl;

$R_{27}$ is $C_6$-$C_{12}$aryl, $C_4$-$C_{12}$heteroaryl, $C_7$-$C_{12}$aralkyl or $C_5$-$C_{12}$heteroaralkyl, each of which is unsubstituted or substituted by one or more, where applicable identical or different, radicals $R_{18}$;

$R_{28}$ is nitro, $SO_2NHR_{25}$,$SO_2NR_{25}R_{26}$, or $C_1$-$C_{12}$alkyl, $C_3$-$C_{12}$cycloalkyl, $C_1$-$C_{12}$alkylthio, $C_3$-$C_{12}$cycloalkylthio, $C_1$-$C_{12}$alkoxy or $C_3$-$C_{12}$cycloalkoxy each unsubstituted or substituted by one or more, where applicable identical or different, radicals $R_{17}$; and

m is a number from 1 to 10.

When $R_5$ forms a bridge with $R_6$, $R_5$ may not at the same time form a bridge with $R_{13}$ or $R_{14}$.

It will be understood that acidic groups, such as carboxy, sulfo, sulfato and phosphato, may also be in the form of a salt, for example an alkali metal, alkaline earth metal, ammonium or phosphonium salt, such as $Li^+$, $Na^+$, $K^+$, $Mg^{2+}$, $Ca^{2+}$, $Cu^{2+}$, $Ni^{2+}$, $Fe^{2+}$, $Co^{2+}$, $Zn^{2+}$, $Sn^{2+}$, $La^{3+}$, ammonium, methylammonium, ethylammonium, isopropylammonium, ™Primene 81-R, ™Rosin Amine D, pentadecylammonium, ™Primene JM-T, dicyclohexylammonium, tetramethylammonium, tetraethylammonium, tetrabutylammonium, benzyltrimethylammonium, benzyltriethylammonium, methyltrioctylammonium, tridodecylmethylammonium, tetrabutylphosphonium, tetraphenylphosphonium, butyltriphenylphosphonium or ethyltriphenylphosphonium, or any of the cations B-1 to B-169 mentioned in US-6 225 024, to which individually reference is expressly made here.

Halogen is chlorine, bromine, fluorine or iodine, preferably fluorine or chlorine, especially fluorine on alkyl (for example trifluoromethyl, $\alpha,\alpha,\alpha$-tritluoroethyl or perfluorinated alkyl groups, such as heptafluoropropyl) and chlorine on aryl, heteroaryl or on the aryl moiety of aralkyl or on the heteroaryl moiety of heteroaralkyl.

Alkyl, cycloalkyl, alkenyl or cycloalkenyl can be straight-chain or branched, or monocyclic or polycyclic. Alkyl is, for example, methyl, straight-chain $C_2$-$C_{24}$alkyl or preferably branched $C_3$-$C_{24}$alkyl. Alkenyl is, for example, straight-chain $C_2$-$C_{20}$alkenyl or preferably branched $C_3$-$C_{24}$alkenyl. The invention therefore relates especially also to compounds of formula (I) containing branched $C_3$-$C_{24}$alkyl or branched $C_3$-$C_{24}$alkenyl, and also to optical recording materials comprising such compounds. $C_1$-$C_{24}$Alkyl is therefore, for example, methyl, ethyl, n-propyl, isopropyl, n-butyl, sec-butyl, isobutyl, tert-butyl, n-pentyl, 2-pentyl, 3-pentyl, 2,2-dimethylpropyl, n-hexyl, n-octyl, 1,1,3,3-tetramethylbutyl, 2-ethylhexyl, nonyl, decyl, dodecyl, tetradecyl, hexadecyl, octadecyl, eicosyl, heneicosyl, docosyl or tetracosyl. $C_3$-$C_{24}$Cycloalkyl is, for example, cydopropyl, cyclobutyl, cyclopentyl, cyclohexyl, trimethylcyclohexyl, menthyl, thujyl, bornyl, 1-adamantyl or 2-adamantyl.

$C_2$-$C_{20}$Alkenyl and $C_3$-$C_{20}$cycloalkenyl are $C_2$-$C_{20}$alkyl and $C_3$-$C_{20}$cycloalkyl that is mono- or poly-unsaturated, wherein two or more double bonds may be isolated or conjugated, for example vinyl, allyl, 2-propen-2-yl, 2-buten-1-yl, 3-buten-1-yl, 1,3-butadien-2-yl, 2-cyclobuten-1-yl, 2-penten-1-yl, 3-penten-2-yl, 2-methyl-1-buten-3-yl, 2-methyl-3-buten-2-yl, 3-methyl-2-buten-1-yl, 1,4-pentadien-3-yl, 2-cyclopenten-1-yl, 2-cyclohexen-1-yl, 3-cyclohexen-1-yl, 2,4-cyclohexadien-1-yl, 1-*p*-menthen-8-yl, 4(10)-thujen-10-yl, 2-norbornen-1-yl, 2,5-norbornadien-1-yl, 7,7-dimethyl-2,4-norcaradien-3-yl or the various isomers of hexenyl, octenyl, nonenyl, decenyl, dodecenyl, tetradecenyl, hexadecenyl, octadecenyl, eicosenyl, heneicosenyl, docosenyl, tetracosenyl, hexadienyl, octadienyl, nonadienyl, decadienyl, dodecadienyl, tetradecadienyl, hexadecadienyl, octadecadienyl or eicosadienyl.

$C_7$-$C_{12}$Aralkyl is, for example, benzyl, 2-benzyl-2-propyl, β-phenyl-ethyl, 9-fluorenyl, α,α-dimethylbenzyl, ω-phenyl-butyl or ω-phenyl-hexyl. When $C_7$-$C_{12}$aralkyl is substituted, both the alkyl moiety and the aryl moiety of the aralkyl group can be substituted, the latter alternative being preferred.

$C_6$-$C_{12}$Aryl is, for example, phenyl, naphthyl, biphenylyl or 2-fluorenyl.

$C_4$-$C_{12}$Heteroaryl is an unsaturated or aromatic radical having $4_{n+2}$ conjugated π-electrons, for example 2-thienyl, 2-furyl, 2-pyridyl, 2-thiazolyl, 2-oxazolyl, 2-imidazolyl, isothiazolyl, triazolyl or any other ring system consisting of thiophene, furan, pyridine, thiazole, oxazole, imidazole, isothiazole, triazole, pyridine and benzene rings and unsubstituted or substituted by from 1 to 6 ethyl, methyl, ethylene and/or methylene substituents, for example benzotriazolyl, and in the case of N-heterocycles where applicable also those in the form of their N-oxides.

$C_5$-$C_{12}$Heteroaralkyl is, for example, $C_1$-$C_8$alkyl substituted by $C_4$-$C_{11}$heteroaryl.

Furthermore, aryl and aralkyl can also be aromatic groups bonded to a metal, for example in the form of metallocenes of transition metals known *per se,* more especially

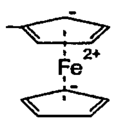 or 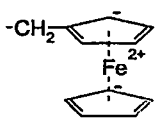 .

The transition metal $M_1$ is preferably in the form of a doubly positively charged cation, for example $Co^{2+}$, $Cu^{2+}$, $Ni^{2+}$, $Pd^{2+}$ or $Zn^{2+}$, especially $Co^{2+}$, $Cu^{2+}$ or $Ni^{2+}$.

The compound of formula (I) may also be a cation which has been neutralised with an inorganic, organic or organometallic anion, for example when one or more ammonium groups are present or when the transition metal has one or more excess positive charges, such as in $Co^{3+}$. The inorganic, organic or organometallic anion may be, for example, the anion of a mineral acid, of the conjugated base of an organic acid (for example an alcoholate, phenolate, carboxylate, sulfonate or phosphonate) or an organometallic complex anion, for example fluoride, chloride, bromide, iodide, perchlorate, periodate, nitrate, hydrogen carbonate, ½ carbonate, ½ sulfate, $C_1$-$C_4$alkyl sulfate, hydrogen sulfate, 1/3 phosphate, ½ hydrogen phosphate, dihydrogen phosphate, ½ $C_1$-$C_4$alkanephosphonate, $C_1$-$C_4$alkane-$C_1$-$C_{12}$alkylphosphonate, di-$C_1$-$C_4$alkylphosphinate, tetrafluoroborate, hexafluorophosphate, hexafluoroantimonate, acetate, trifluoroacetate, heptafluorobutyrate, ½ oxalate, methanesulfonate, trifluoromethanesulfonate, benzenesulfonate, tosylate, p-chlorobenzenesulfonate, p-nitrobenzenesulfonate, phenolate, benzoate or a negatively charged metal complex.

The person skilled in the art will readily recognise that it is also possible to use other anions with which he is familiar. It will be self-evident to him that $\frac{1}{x}$ of an inorganic, organic or organometallic anion having x negative charges, for example ½· $SO_4^{2-}$, is a multiply charged anion which neutralises several singly charged cations or a cation having x charges, as the case may be.

Phenolates or carboxylates are, for example, of formula

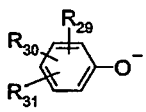 or 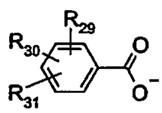

(wherein $R_{29}$, $R_{30}$ and $R_{31}$ are each independently of the others hydrogen, $R_{18}$, or $C_6$-$C_{12}$aryl, $C_4$-$C_{12}$heteroaryl, $C_7$-$C_{12}$aralkyl or $C_5$-$C_{12}$heteroaralkyl each unsubstituted or substituted by one or more, where applicable identical or different, radicals $R_{18,}$ for example anions of $C_1$-$C_{12}$alkylated, especially tert-$C_4$-$C_8$alkylated, phenols and benzoic acids, such as

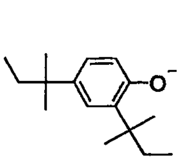 , 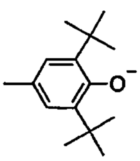 or 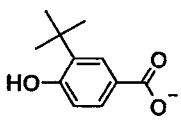 .

Preference is given to compounds of formula (I) wherein
$A_1$, $A_2$ and $A_3$ are each independently of the others O, S or $N(R_{12})$ and/or $Q_1$ and $Q_2$ are $C(R_{15})$ or N;
$G_1$ and $G_2$ are each independently of the other

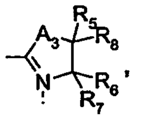,
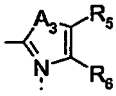

or

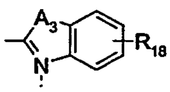, especially 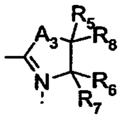 or 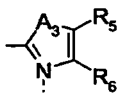;

$R_1$, $R_2$, $R_3$, $R_4$, $R_5$, $R_6$, $R_7$, $R_8$ and $R_{14}$ are each independently of the others hydrogen, $R_{18}$, or $C_6$-$C_{12}$aryl or $C_7$-$C_{12}$aralkyl each unsubstituted or substituted by one or more, where applicable identical or different, radicals $R_{18}$;
$R_9$, $R_{12}$ and $R_{13}$ are each independently of the others unsubstituted or $R_{17}$-substituted $C_1$-$C_8$alkyl;
$R_{10}$ and $R_{18}$ are each independently of the other halogen, nitro, cyano, O-$R_{19}$, formyl, $CH=C(CN)_2$, $CH=C(CN)CONH_2$, $CH=C(CN)CONHR_{19}$, $CH=C(CN)CONR_{19}R_{20}$, $CH=C(CN)COOR_{19}$, $CH=C(COOR_{19})COOR_{20}$, $CONH_2$, $CONHR_{19}$, $CONR_{19}R_{20}$, $SO_2C_1$-$C_{12}$alkyl, $SO_2NH_2$, $SO_2NHR_{19}$, $SO_2NR_{19}R_{20}$, COOH, $COOR_{19}$, $NHCOR_{19}$, $NR_{19}COR_{23}$, $NHCOOR_{19}$, $NR_{19}COOR_{23}$, ureido, $P(=O)OR_{19}OR_{23}$, sulfo, or $C_1$-$C_{12}$alkyl, $C_1$-$C_{12}$alkylthio or $C_1$-$C_{12}$allioxy each unsubstituted or substituted by one or more, where applicable identical or different, radicals $R_{17}$;
$R_{15}$ is hydrogen, cyano, halogen, nitro, formyl, N=N-$R_{27}$, $C(R_{14})=CR_{21}R_{22}$, $C(R_{14})=NR_{19}$, COO-$R_{25}$, carboxy, carbamoyl, CONH-$R_{25}$, $CONR_{25}R_{26}$, or $C_1$-$C_{12}$alkyl unsubstituted or substituted by one or more halogen substituents;
$R_{16}$ is unsubstituted or substituted $C_6$-$C_{12}$aryl or $C_7$-$C_{12}$aralkyl, especially a metallocenyl radical;
$R_{17}$ is halogen, hydroxy, O-$R_{25}$, amino, NH-$R_{25}$, $NR_{25}R_{26}$, $NR_{25}$-CO-$R_{24}$, $NR_{25}COOR_{24}$, cyano, COO-$R_{25}$, carboxy, CONH-$R_{25}$, $CONR_{25}R_{26}$, sulfato, sulfo, or $C_1$-$C_{12}$alkoxy unsubstituted or mono- or poly-substituted by halogen;
$R_{19}$, $R_{20}$ and $R_{23}$ are each independently of the others $C_1$-$C_{12}$alkyl unsubstituted or substituted by one or more, where applicable identical or different, halogen, hydroxy or $C_1$-$C_{12}$alkoxy radicals, or unsubstituted $C_6$-$C_{12}$aryl or $C_7$-$C_{12}$aralkyl; or
$R_{19}$ and $R_{20}$ together with the common nitrogen are morpholine, or piperidine N-substituted by $C_1$-$C_4$alkyl;
$R_{25}$, $R_{26}$ and $R_{24}$, are each independently of the others $C_1$-$C_{12}$alkyl, $C_2$-$C_{12}$alkenyl, $C_6$-$C_{12}$aryl or $C_7$-$C_{12}$aralkyl; or
$R_{25}$ and $R_{26}$ together with the common nitrogen are morpholine, or piperidine N-substituted by $C_1$-$C_4$alkyl; and/or
m is a number from 1 to 4.

Special preference is given to compounds of formula (I) wherein $Q_1$ and $Q_2$ are $C(R_{15})$; $G_1$ and $G_2$ are

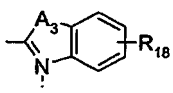;

and $A_1$, $A_2$ and $A_3$ are O, S or $N(R_{12})$;

$R_{12}$ is $C_1$-$C_{24}$alkyl, $C_1$-$C_4$alkyl-[O-$C_1$-$C_4$alkylene]$_m$ or $C_1$-$C_4$alkyl-[NH-$C_1$-$C_4$alkylene]$_m$, each of which is unsubstituted or substituted by one or more, where applicable identical or different, radicals $R_{17}$, or $C_6$-$C_{12}$aryl unsubstituted or substituted by one or more, where applicable identical or different, radicals $R_{18}$;
$R_{15}$ is hydrogen, cyano, COO-$R_{25}$ or $C_1$-$C_{12}$alkyl;
$R_{17}$ is halogen, hydroxy, O-$R_{25}$, cyano, COO-$R_{25}$ or carboxy; and
$R_{18}$ is halogen, nitro, cyano, O-$R_{19}$, $CH=C(CN)_2$, $COOR_{19}$, ureido, $CONR_{25}R_{26}$, $SO_2R_{25}$, $P(=O)OR_{19}OR_{23}$ or unsubstituted or substituted $C_1$-$C_{12}$alkyl.

Those preferred meanings apply both individually and in any combination. The compounds of formula (I) generally exhibit more advantageous properties, the more preferred individual features they have.

Also preferred are compounds of formula (I) wherein

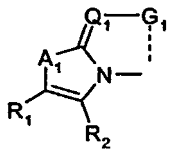

and/or

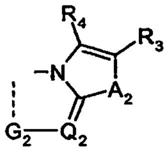 is/are 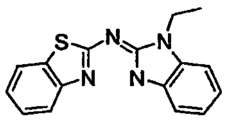, 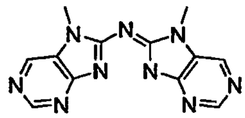,

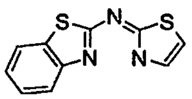, 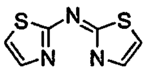, 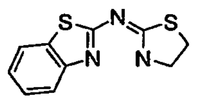, 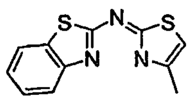,

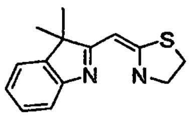, 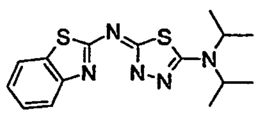, 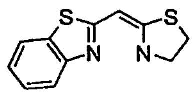, 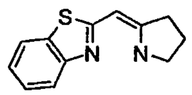,

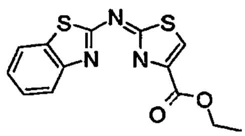 or 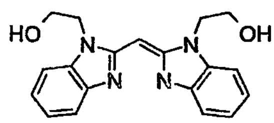.

The recording layer advantageously comprises a compound of formula (I) or a mixture of such compounds as main component, for example at least 30 % by weight, preferably at least 60 % by weight, especially at least 80 % by weight Further customary constituents are possible, for example other chromophores (for example those disclosed in WO 01/75873, or others having an absorption maximum at from 300 to 1000 nm), stabilisers, $^1O_2$-, triplet- or luminescence-quenchers, melting-point reducers, decomposition accelerators or any other additives that have already been described in optical recording media. Preferably, stabilisers or fluoresence-quenchers are added if desired.

When the recording layer comprises further chromophores, the amount of such chromophores should preferably be small, so that the absorption thereof at the wavelength of the inversion point of the longest-wavelength flank of the absorption of the entire solid layer is a fraction of the absorption of the pure compound of formula (I) in the entire solid layer at the same wavelength, advantageously at most 1/3, preferably at most 1/5, especially at most 1/10. The absorption maximum is preferably higher than 425 nm, especially higher than 500 nm.

Stabilisers, $^1O_2$-, triplet- or luminescence-quenchers are, for example, metal complexes of N- or S-containing enolates, phenolates, bisphenolates, thiolates or bisthiolates or of azo, azomethine or formazan dyes, such as bis(4-dimethylaminodithiobenzil)nickel [CAS N° 38465-55-3], ®Irgalan Bordeaux EL, ®Cibafast N or similar compounds, hindered phenols and derivatives thereof (optionally also as counter-ions X), such as ®Cibafast AO, o-hydroxyphenyl-triazoles or -triazines or other UV absorbers, such as ®Cibafast W or ®Cibafast P or hindered amines (TEMPO or HALS, also as nitroxides or NOR-HALS, optionally also as counter-ions X), and also as cations diimmonium, Paraquat™ or Orthoquat™ salts, such as ®Kayasorb IRG 022, ®Kayasorb IRG 040, optionally also as radical ions, such as N,N,N',N'-tetrakis(4-dibutylaminophenyl)-p-phenyleneamine-ammonium hexafluorophosphate, hexafluoroantimonate or perchlorate. The latter are available from Organica (Wolfen / DE); ®Kayasorb brands are available from Nippon Kayaku Co. Ltd., and ®Irgalan and ®Cibafast brands are available from Ciba Spezialitätenchemie AG.

Many such structures are known, some of them also in connection with optical recording media, for example

from US-5 219 707, JP-A-06/199045, JP-A-07/76169, JP-A-07/262604 or JP-A-2000/272241. They may be, for example, salts of the metal complex anions disclosed above with any desired cations, for example the cations disclosed above, or metal complexes, illustrated, for example, by a compound of formula

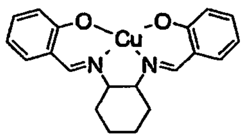 or 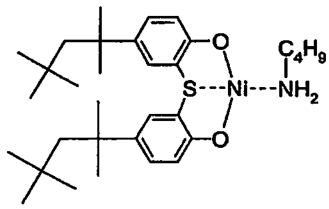 .

The person skilled in the art will know from other optical information media, or will easily identify, which additives in which concentration are particularly well suited to which purpose. Suitable concentrations of additives are, for example, from 0.001 to 1000% by weight, preferably from 1 to 50% by weight, based on the recording medium of formula (I).

The optical recording materials according to the invention exhibit excellent spectral properties of the solid amorphous recording layer. The refractive index is extraordinarily high, in some cases even above 2.5. By virtue of an aggregation tendency in the solid that is surprisingly low for such compounds, the absorption band is narrow and intense, the absorption band being especially steep on the long-wavelength side. Crystallites are unexpectedly and very advantageously not formed or are formed only to a negligible extent. The reflectivity of the layers in the range of the writing and reading wavelength is very high in the unwritten state.

By virtue of those excellent layer properties it is possible to obtain a rapid optical recording having high sensitivity, high reproducibility and geometrically very precise mark boundaries, the refractive index and the reflectivity changing substantially, which gives a high degree of contrast. The differences in the mark lengths and the interval distances ("jitter") are very small, which enables a high storage density to be obtained using a relatively thin recording channel with a narrow track spacing ("pitch"). In addition, the recorded data are played back with an astonishingly low error rate, so that error correction requires only a small amount of storage space. By virtue of the excellent solubility, including in apolar solvents, solutions can be used even in high concentrations without troublesome precipitation, for example during storage, so that problems during spin-coating are largely eliminated. This applies especially to compounds containing branched $C_3$-$C_8$alkyl.

Recording and playback can take place at the same wavelength, therefore advantageously requiring a simple optical system with a single laser source of advantageously from 350 to 500 nm, preferably from 370 to 450 nm. Especially preferred is the UV range from 370 to 390 nm, especially approximately 380 nm, or especially at the edge of the visible range of from 390 to 430 nm, more especially approximately 405 $\pm$5 nm. In the field of compact, blue or violet laser diodes (such as Nichia GaN 405 nm) with an optical system of high numerical aperture the marks can be so small and the tracks so narrow that up to about 20 to 25 Gb per recording layer is achievable on a 120 mm disc. At 380 nm it is possible to use indium-doped UV-VCSELs (Vertical-Cavity Surface-Emitting Laser), which laser source already exists as a prototype [Jung Han *et al.,* see MRS Internet J. Nitride Semicond. Res. 5S1, W6.2 (2000)].

The invention therefore relates also to a method of recording or playing back data, wherein the data on an optical recording medium according to the invention are recorded or played back at a wavelength of from 350 to 500 nm.

The recording medium is based on the structure of known recording media and is, for example, analogous to those mentioned above. It may be composed, for example, of a transparent substrate, a recording layer comprising at least one of the compounds of formula (I), a reflector layer and a covering layer, the writing and readout being effected through the substrate.

Suitable substrates are, for example, glass, minerals, ceramics and thermosetting and thermoplastic plastics. Preferred supports are glass and homo- or co-polymeric plastics. Suitable plastics are, for example, thermoplastic polycarbonates, polyamides, polyesters, polyacrylates and polymethacrylates, polyurethanes, polyolefins, polyvinyl chloride, polyvinylidene fluoride, polyimides, thermosetting polyesters and epoxy resins. Special preference is given to polycarbonate substrates which can be produced, for example, by injection-moulding. The substrate can be in pure form or may comprise customary additives, for example UV absorbers or dyes, as proposed e.g. in JP-A-04/167239 as light stabilisation for the recording layer. In the latter case it may be that in the range of the writing wavelength (emission wavelength of the laser) the dye added to the support substrate has no or at most only very low absorption, preferably up to a maximum of about 20% of the laser light focussed onto the recording layer.

The substrate is advantageously transparent over at least a portion of the range from 350 to 500 nm, so that it is permeable to, for example, at least 80 % of the incident light of the writing or readout wavelength. The substrate is advantageously from 10 $\mu$m to 2 mm thick, preferably from 100 to 1200 $\mu$m thick, especially from 600 to 1100 $\mu$m thick,

with a preferably spiral guide groove (track) on the coating side, a groove depth of from 10 to 200 nm, preferably from 80 to 150 nm, a groove width of from 100 to 400 nm, preferably from 150 to 250 nm, and a spacing between two turns of from 200 to 600 nm, preferably from 350 to 450 nm. Grooves of different cross-sectional shape are known, for example rectangular, trapezoidal or V-shaped. Analogously to the known CD-R and DVD-R media, the guide groove may additionally undergo a small periodic or quasi-periodic lateral deflection (wobble), so that synchronisation of the speed of rotation and the absolute positioning of the reading head (pick-up) are made possible. Instead of, or in addition to, the deflection, the same function can be performed by markings between adjacent grooves (pre-pits).

The recording medium is applied, for example, by application of a solution by spin-coating, the objective being to produce a layer that is as amorphous as possible, the thickness of which layer is advantageously from 0 to 40 nm, preferably from 1 to 20 nm, especially from 2 to 10 nm, on the surface ("land") and, depending upon the geometry of the groove, advantageously from 20 to 150 nm, preferably from 50 to 120 nm, especially from 60 to 100 nm, in the groove.

Reflecting materials suitable for the reflector layer include especially metals, which provide good reflection of the laser radiation used for recording and playback, for example the metals of Main Groups III, IV and V and of the Sub-Groups of the Periodic Table of the Elements. Al, In, Sn, Pb, Sb, Bi, Cu, Ag, Au, Zn, Cd, Hg, Sc, Y, La, Ti, Zr, Hf, V, Nb, Ta, Cr, Mo, W, Fe, Co, Ni, Ru, Rh, Pd, Os, Ir, Pt and the lanthanide metals Ce, Pr, Nd, Pm, Sm, Eu, Gd, Tb, Dy, Ho, Er, Tm, Yb and Lu and alloys thereof are especially suitable. On account of its high reflectivity and ease of production special preference is given to a reflective layer of aluminium, silver, gold or an alloy thereof (for example a white gold alloy), especially aluminium on economic and ecological grounds. The reflector layer is advantageously from 5 to 200 nm thick, preferably from 10 to 100 nm thick, especially from 40 to 60 nm thick, but reflector layers of greater thickness, for example 1 mm thick or even more, are also possible.

Materials suitable for the covering layer include chiefly plastics, which are applied in a thin layer to the reflector layer either directly or with the aid of adhesion promoters. It is advantageous to select mechanically and thermally stable plastics having good surface properties, which can be modified further, for example written on. The plastics may be thermosetting plastics and thermoplastic plastics. Directly applied covering layers are preferably radiation-cured (e.g. using UV radiation) coatings, which are particularly simple and economical to produce. A wide variety of radiation-curable materials are known. Examples of radiation-curable monomers and oligomers are acrylates and methacrylates of diols, triols and tetrols, polyimides of aromatic tetracarboxylic acids and aromatic diamines having $C_1$-$C_4$alkyl groups in at least two ortho-positions of the amino groups, and oligomers with dialkylmaleinimidyl groups, e.g. dimethylmaleinimidyl groups. For covering layers that are applied using adhesion promoters it is preferable to use the same materials as those used for the substrate layer, especially polycarbonates. The adhesion promoters used are preferably likewise radiation-curable monomers and oligomers. Instead of the covering layer applied using an adhesion promoter there may also be used a second substrate comprising a recording and reflector layer, so that the recording medium is playable on both sides. Preference is given to a symmetrical structure, the two parts being joined together at the reflector side by an adhesion promoter directly or by way of an intermediate layer.

In such a structure, the optical properties of the covering layer, or the covering materials, are essentially unimportant *per se* provided that, where applicable, curing thereof e.g. by UV radiation is achieved. The function of the covering layer is to ensure the mechanical strength of the recording medium as a whole and, if necessary, the mechanical strength of thin reflector layers. If the recording medium is sufficiently robust, for example when a thick reflector layer is present, it is even possible to dispense with the covering layer altogether. The thickness of the covering layer depends upon the thickness of the recording medium as a whole, which should preferably be a maximum of about 2 mm thick. The covering layer is preferably from 10 $\mu$m to 1 mm thick.

The recording media according to the invention may also have additional layers, for example interference layers or barrier layers. It is also possible to construct recording media having a plurality of (for example from two to ten) recording layers. The structure and the use of such materials are known to the person skilled in the art. Where present, interference layers are preferably arranged between the recording layer and the reflecting layer and/or between the recording layer and the substrate and consist of a dielectric material, for example as described in EP-A-0 353 393 of $TiO_2$, $Si_3N_4$, ZnS or silicone resins.

The recording media according to the invention can be produced by processes known *per se,* it being possible for various methods of coating to be employed depending upon the materials used and their function.

Suitable coating methods are, for example, immersion, pouring, brush-coating, blade-application and spin-coating, as well as vapour-deposition methods carried out under a high vacuum. When, for example, pouring methods are used, solutions in organic solvents are generally employed. When solvents are employed, care should be taken that the supports used are insensitive to those solvents. Suitable coating methods and solvents are described, for example, in EP-A-0 401 791.

The recording layer is applied preferably by the application of a dye solution by spin-coating, solvents that have proved satisfactory being especially alcohols, e.g. 2-methoxyethanol, isopropanol or n-butanol, hydroxyketones, for example diacetone alcohol or 3-hydroxy-3-methyl-2-butanone, hydroxy esters, for example lactic acid methyl ester or isobutyric acid methyl ester, or preferably fluorinated alcohols, for example 2,2,2-trifluoroethanol or 2,2,3,3-tetrafluoro-1-propanol, and mixtures thereof. Further suitable solvents are disclosed, for example, in EP-A-0 483 387.

The application of the metallic reflector layer is preferably effected by sputtering or by vapour-deposition *in vacuo*. Such techniques are known and are described in specialist literature (e.g. J.L. Vossen and W. Kem, "Thin Film Processes", Academic Press, 1978). The operation can advantageously be carried out continuously and achieves good reflectivity and a high degree of adhesiveness of the metallic reflector layer.

Recording is carried out in accordance with known methods by writing pits (marks) of fixed or variable length by means of a modulated, focussed laser beam guided at a constant or variable speed over the surface of the recording layer. Readout of information is carried out according to methods known *per se* by registering the change in reflection using laser radiation, for example as described in "CD-Player und R-DAT Recorder" (Claus Biaesch-Wiepke, Vogel Buchverlag, Würzburg 1992).The person skilled in the art will be familiar with the requirements.

The information-containing medium according to the invention is especially an optical information material of the WORM type. It can be used, for example, analogously to CD-R (compact disc - recordable) or DVD-R (digital video disc - recordable) in computers, and also as storage material for identification and security cards or for the production of diffractive optical elements, for example holograms.

Alternatively, however, there are also recording media which differ substantially from CD-R and DVD-R and in which recording and playback take place not through the substrate but through the covering layer ("in-groove recording"). Accordingly the respective roles of the covering layer and the substrate, especially the geometry and the optical properties, are reversed in comparison with the structure described above. Analogous concepts are described a number of times in Proceedings SPIE-Int Soc. Opt. Eng. 1999, 3864 for digital video recordings in conjunction with a blue GaN laser diode. For such recording media, which are especially suitable for a high storage density and have correspondingly small marks ("pits"), precise focussing is important, so that the manufacturing process, while essentially analogous, is considerably more awkward.

The compounds of formula (I) according to the invention, however, also meet the increased demands of an inverse layer structure surprisingly well. Preference is therefore given to an inverse layer structure having the layer sequence substrate, reflector layer, recording layer and covering layer. The recording layer is therefore located between the reflector layer and the covering layer. A thin covering layer approximately from 50 to 400 μm in thickness is especially advantageous (typically 100 μm at a numerical aperture of 0.85).

The recording and reflector layers in an inverse layer structure have in principle the same functions as indicated above. As with the groove geometry, they therefore usually have dimensions within the ranges indicated above.

The inverse layer structure requires particularly high standards, which the compounds used according to the invention fulfil astonishingly well, for example when the recording layer is applied to the metallic reflector layer and especially when a covering layer is applied to the recording layer, the covering layer being required to provide the recording layer with adequate protection against rubbing, photooxidation, fingerprints, moisture and other environmental effects and advantageously having a thickness in the range of from 0.01 to 0.5 mm, preferably in the range of from 0.05 to 0.2 mm, especially in the range of from 0.08 to 0.13 mm.

The covering layer preferably consists of a material that exhibits a transmission of 80% or above at the writing or readout wavelength of the laser. Suitable materials for the covering layer include, for example, those materials mentioned above, but especially polycarbonate (such as Pure Ace® or Panlite®, Teijin Ltd), cellulose triacetate (such as Fujitac®, Fuji Photo Film) or polyethylene terephthalate (such as Lumirror®, Toray Industry), special preference being given to polycarbonate. Especially in the case of directly applied covering layers, radiation-cured coatings, such as those already described above, are advantageous, for example SD 347™ (Dainippon Ink).

The covering layer can be applied directly to the solid recording layer by means of a suitable adhesion promoter. In another embodiment, there is applied to the solid recording layer an additional, thin separating layer of a metallic, crosslinked organometallic or preferably dielectric inorganic material, for example in a thickness of from 0.001 to 10 μm, preferably from 0.005 to 1 μm, especially from 0.01 to 0.1 μm, for example from 0.05 to 0.08 μm in the case of dielectric separating layers and from 0.01 to 0.03 μm in the case of metallic separating layers. Separating layers and corresponding methods are disclosed in WO 02/082438, to which reference is expressly made here. If desired, such coatings can be applied, for example, in the same thickness also between the support material and the metallic reflector layer or between the metallic reflector layer and the optical recording layer. This may be advantageous in certain cases, for example when a silver reflector is used in combination with sulfur-containing additives in the recording layer.

In a special variant, there is applied to the solid recording layer an additional, thin separating layer of a metallic, crosslinked organometallic or dielectric inorganic material, for example in a thickness of from 0.001 to 10 μm, preferably from 0.005 to 1 μm, especially from 0.01 to 0.1 μm. On account of their high reflectivity, metallic separating layers should advantageously be a maximum of 0.03 μm thick.

Separating layers and corresponding methods are disclosed in WO 02/082438, to which reference is expressly made here.

Some of the compounds used according to the invention are known, especially from J. Org. Chem. 67/16, 5753-5772 [2002].

It is also possible, however, to prepare analogously to the known compounds new compounds that can be used in accordance with the invention in optical recording media.

The invention therefore relates also to compounds of formula (I), with the exception of the already known compounds of formula $M_2(Z_1)_2$, wherein:

- $M_2$ is Co(II), Cu(II), Hg(II), Ni(II), Pd(II) or Zn(II) and $Z_1$ is

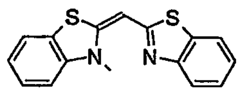 ;

- $M_2$ is Co(II), Cu(II), Ni(II), Pd(II) or Zn(II) and $Z_1$ is

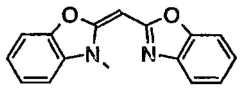 ,

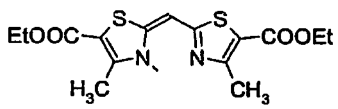 , 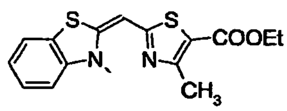 ,

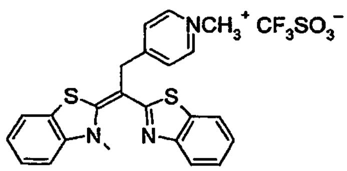 or 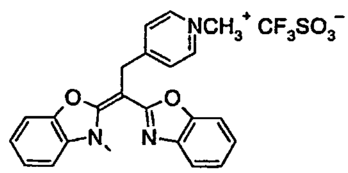 ;

- $M_2$ is Co(II), Cu(II), Ni(II) or Zn(II) and $Z_1$ is

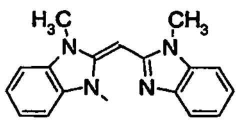 ;

- $M_2$ is Cu(II), Pd(II) or Zn(II) and $Z_1$ is

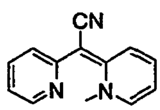 ;

- $M_2$ is Co(II), Cu(II) or Zn(II) and $Z_1$ is

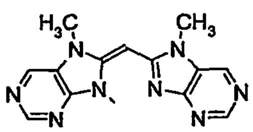 or 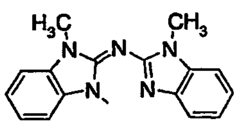 ;

- $M_2$ is Cu(II) or Zn(II) and $Z_1$ is

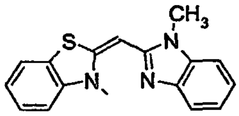 , 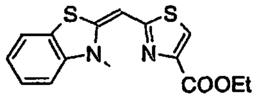 or 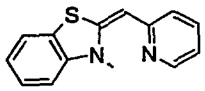 ;

- $M_2$ is Co(II) or Cu(II) and $Z_1$ is

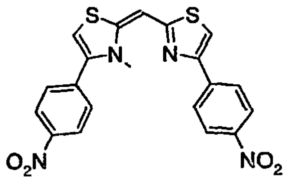 ;

- $M_2$ is Pd(II) or Zn(II) and $Z_1$ is

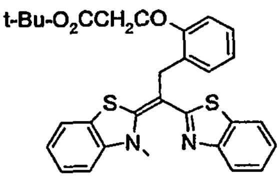 or 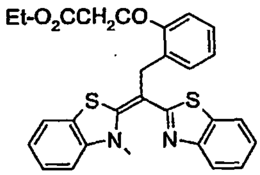 ;

- $M_2$ is Cu(II) and $Z_1$ is

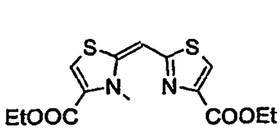 or 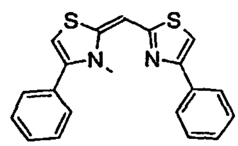 ;

or

- $M_2$ is Zn(II) and $Z_1$ is

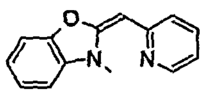 or 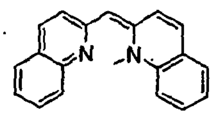 .

Reference is made especially to compounds of formulae

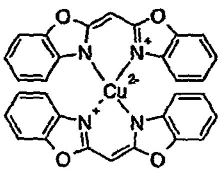 , 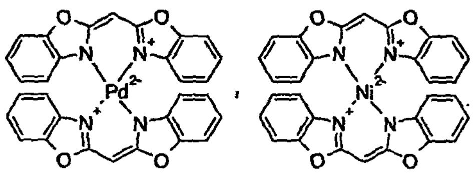 ,

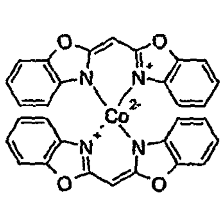, 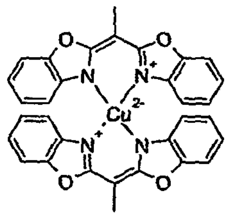, 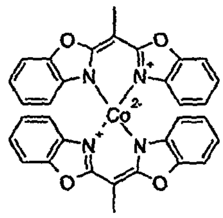,

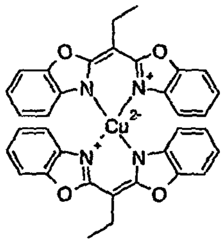, 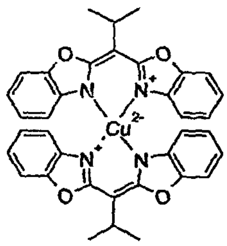, 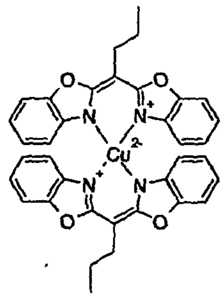,

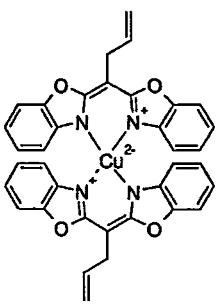, 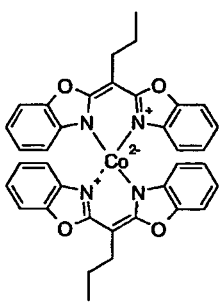, 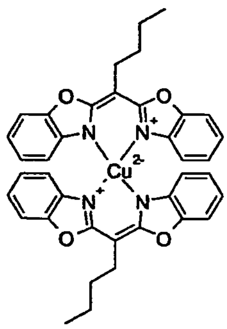,

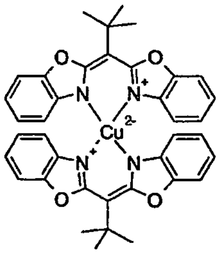, 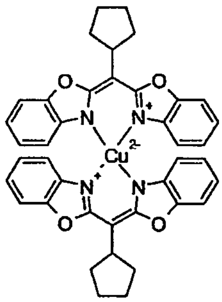, 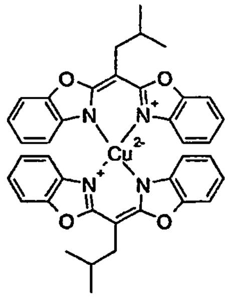,

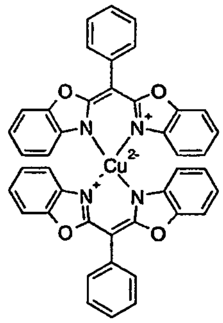 , 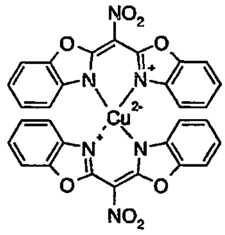 , 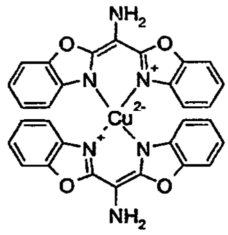 ,

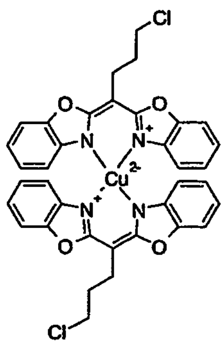 , 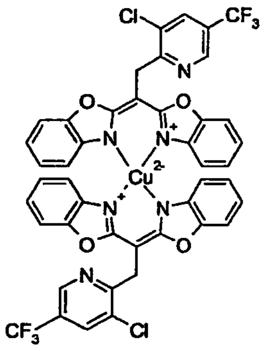 , 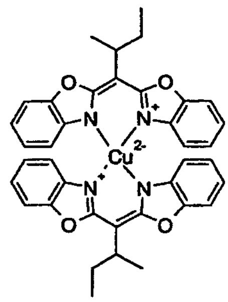 ,

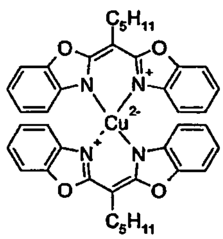 , 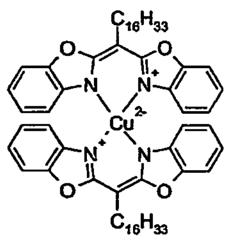 , 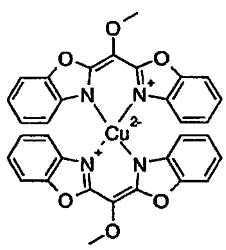 ,

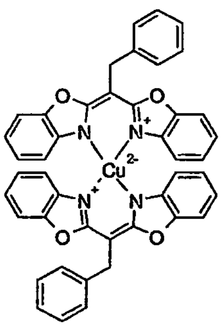 and 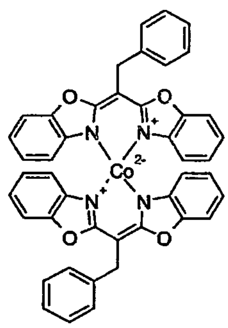 ,

.

and to compounds wherein $M_2$ is Cu, Co, Ni or Pd and $Z_1$ is a radical of the following compounds:

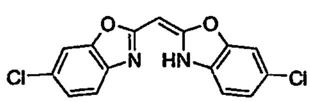 , 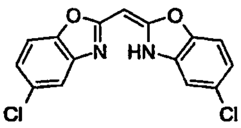 , 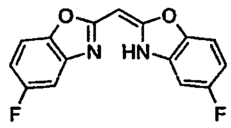 ,

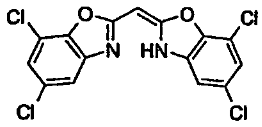 , 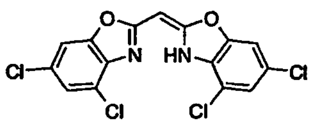 , 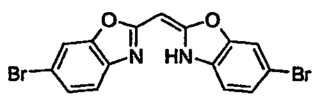 ,

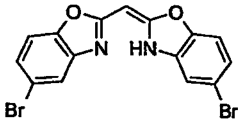 , 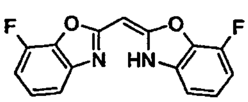 ,

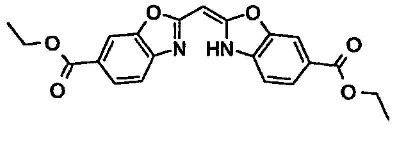 , 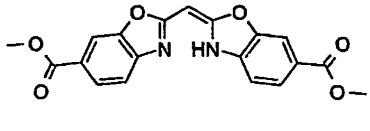 ,

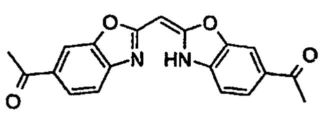 , 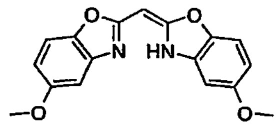 ,

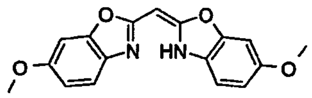 , 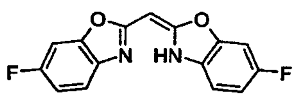 , 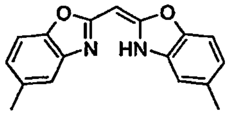 ,

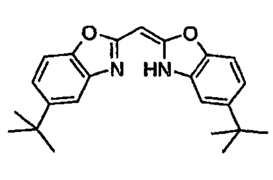 , 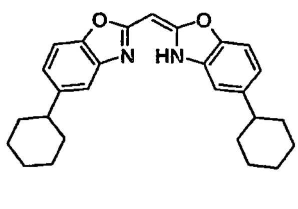 , 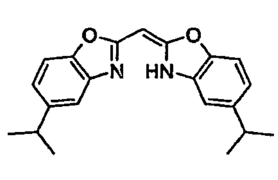 ,

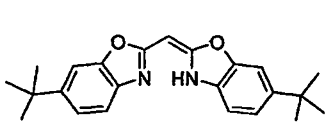 , 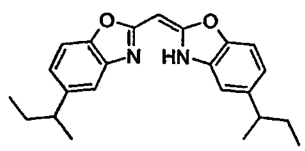 ,

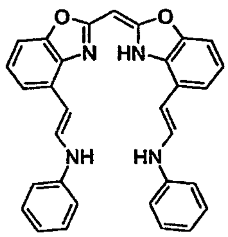 , 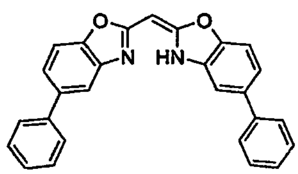 , 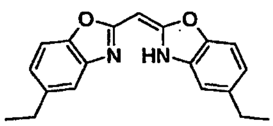 ,

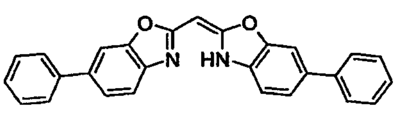 , 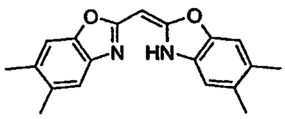 ,

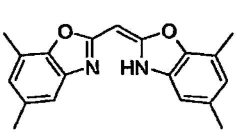 , 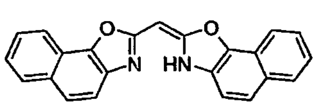 ,

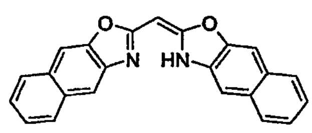 , 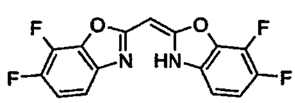 , 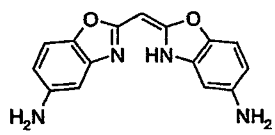 ,

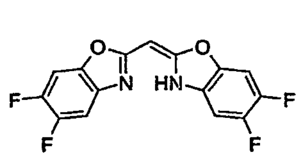 , 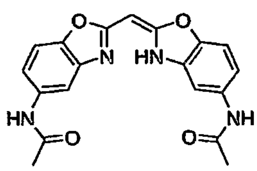 , 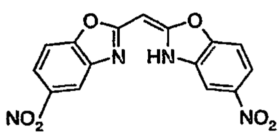 ,

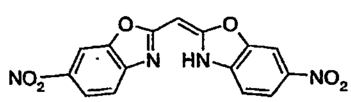 , 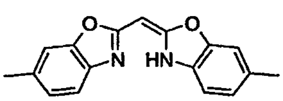 ,

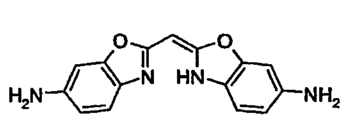 , 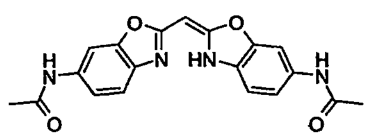 ,

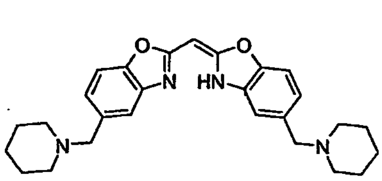 , 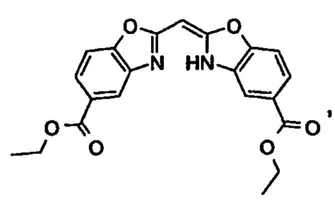 ,

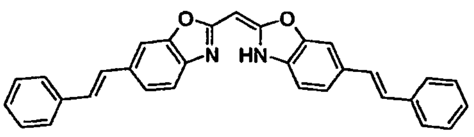 , 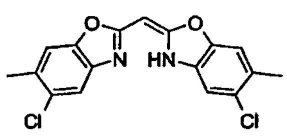 ,

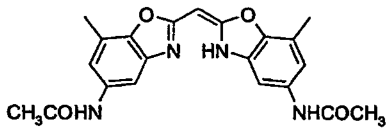 , 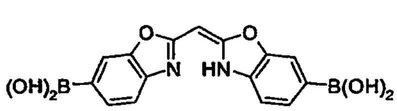 ,

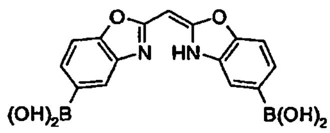 , 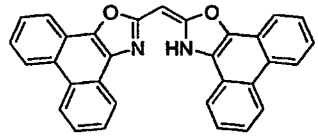 ,

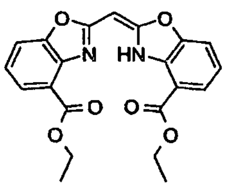 , 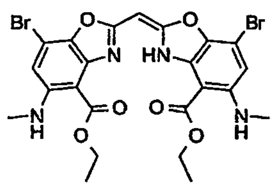 , 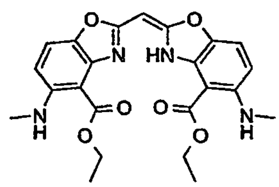 ,

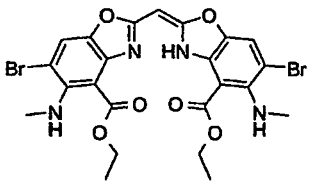 , 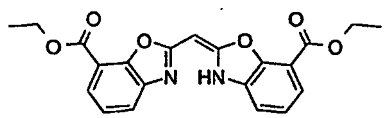 ,

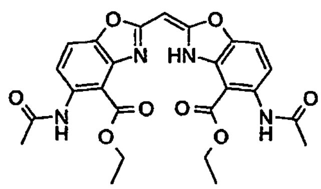 , 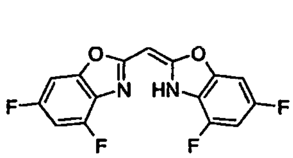 ,

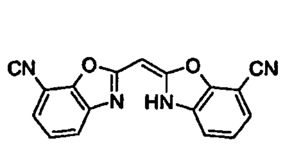 , 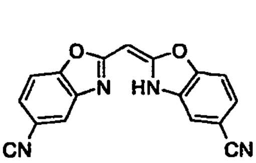 , 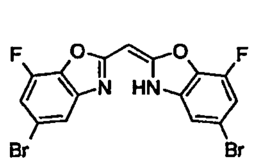 ,

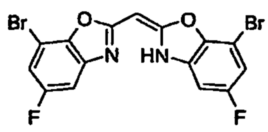
,
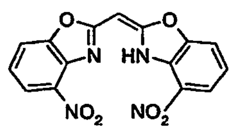
,
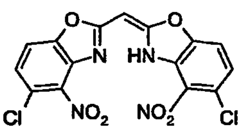
,

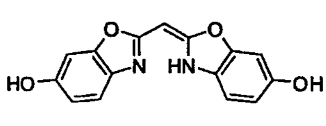
,
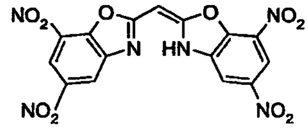
,
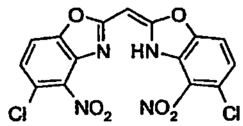
,

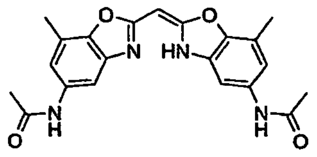
,
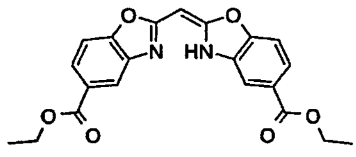
,

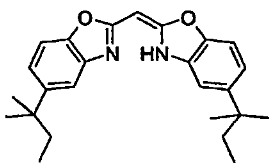
,
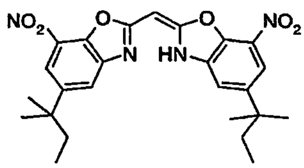
,
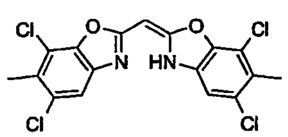
,

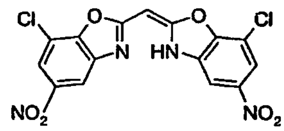
,
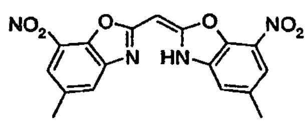
,

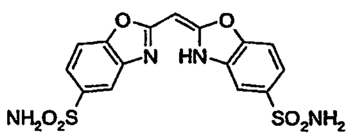
,
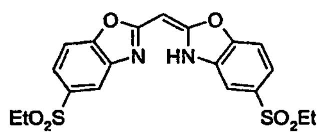
,

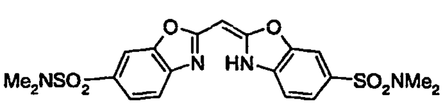
,
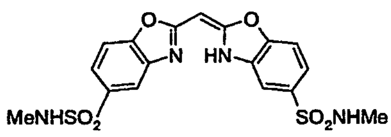
,

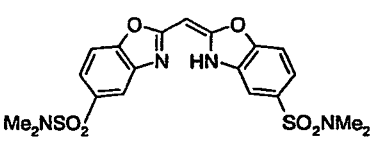
,
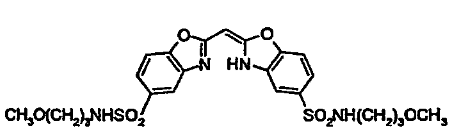
,

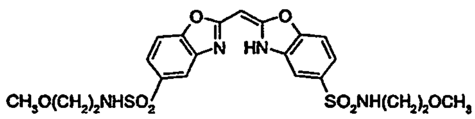,

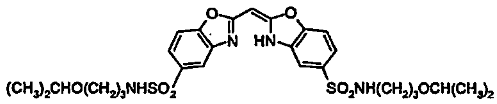,

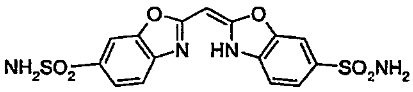, 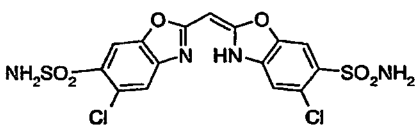,

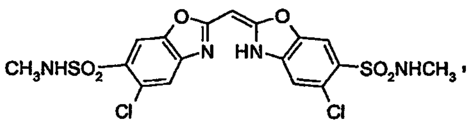, 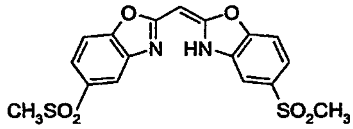,

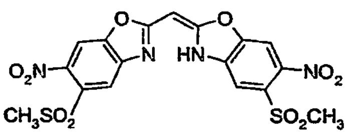, 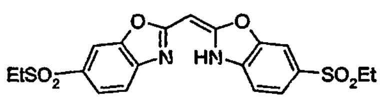,

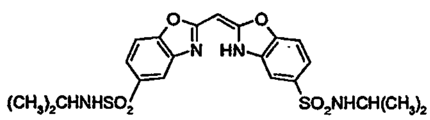, 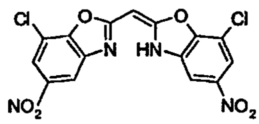,

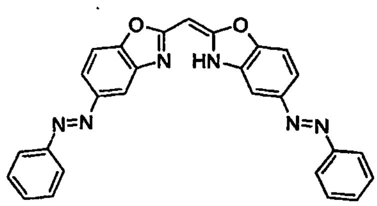, 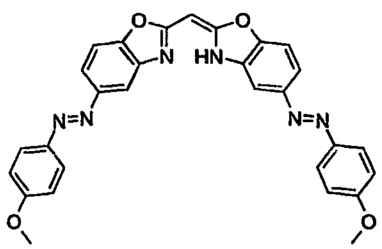,

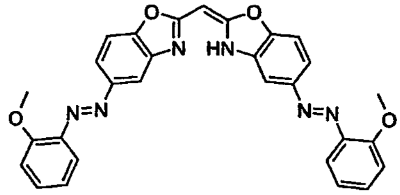, 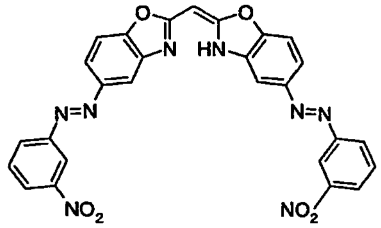,

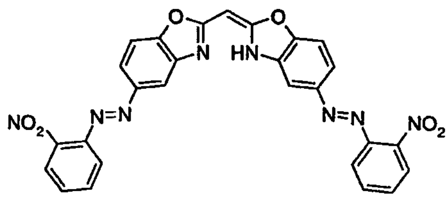,
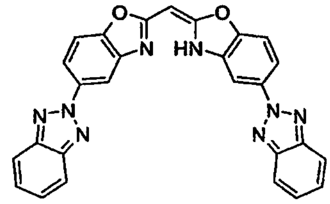,

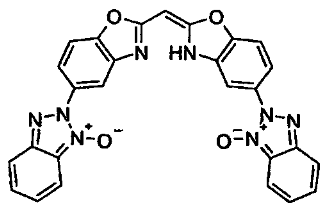,
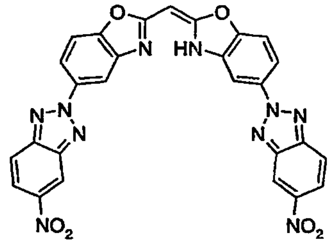,

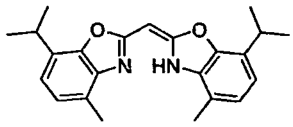,
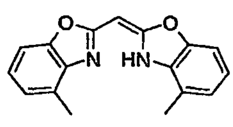,
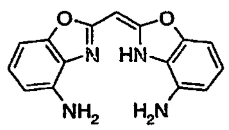,

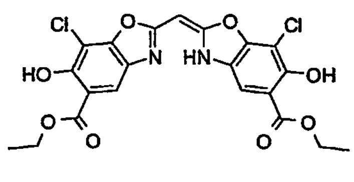,
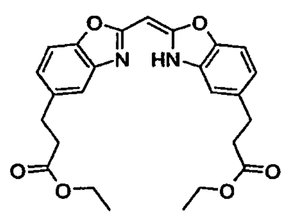,

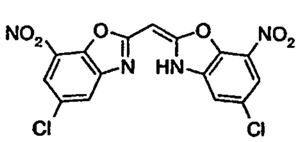,
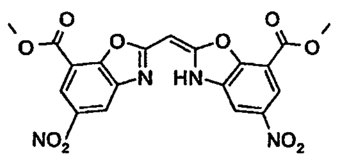,
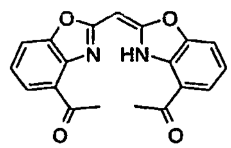,

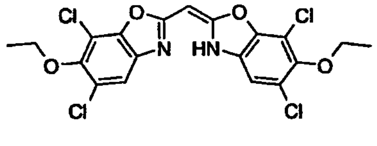,
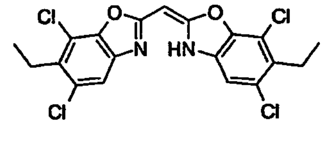,

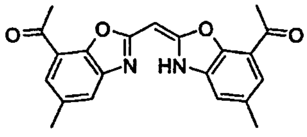,
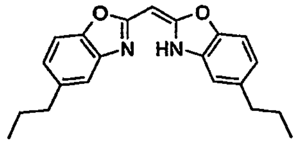,

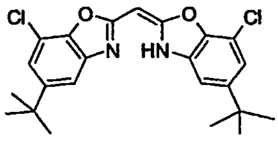, 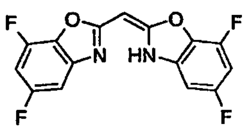, 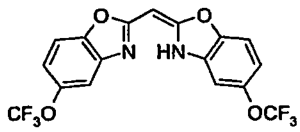,

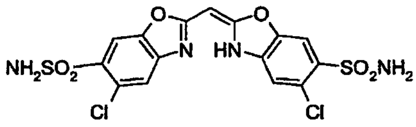, 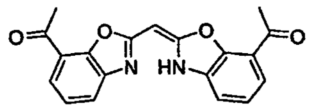,

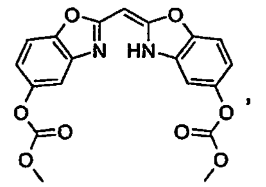, 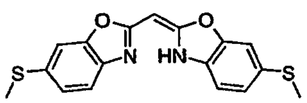,

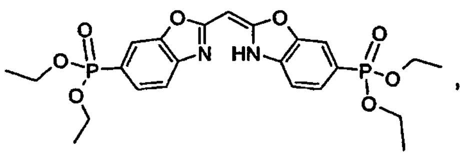, 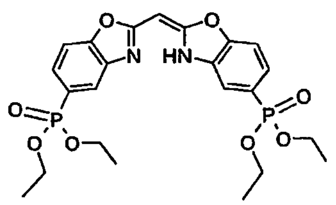,

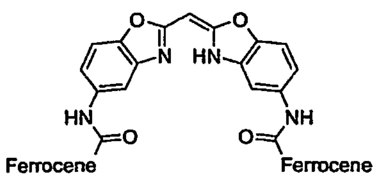, 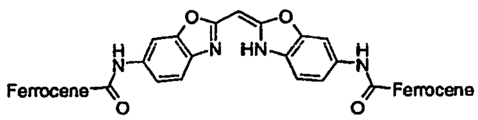,

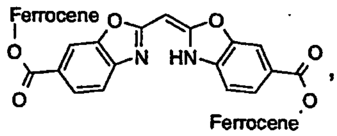, 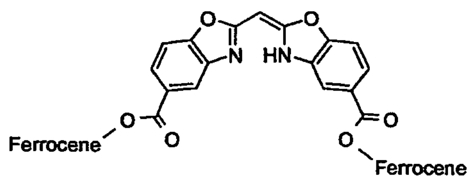,

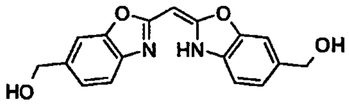, 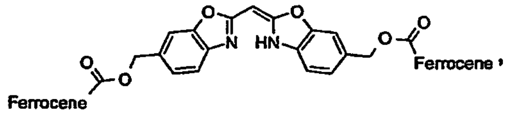,

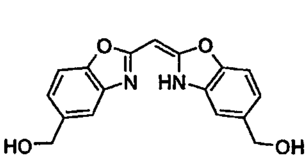,
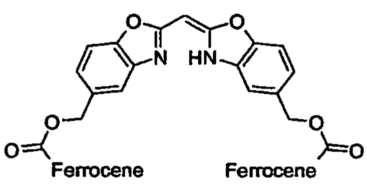,

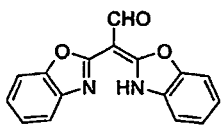,
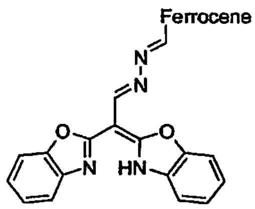,
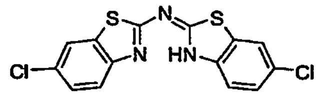,

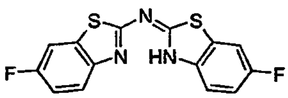,
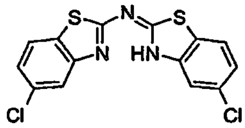,
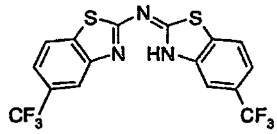,

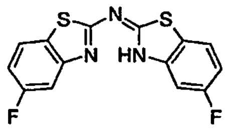,
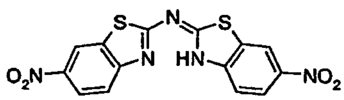,
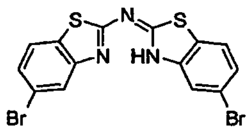,

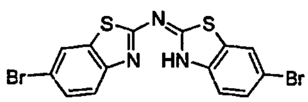,
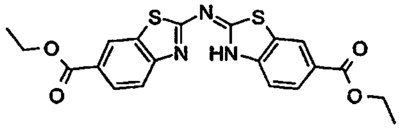,

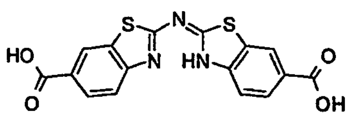,
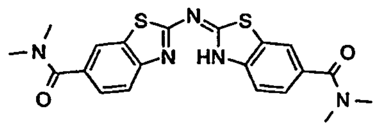,

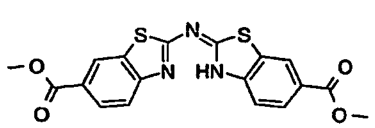,
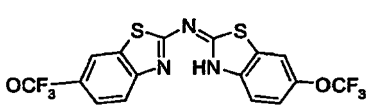,

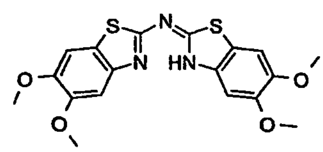,
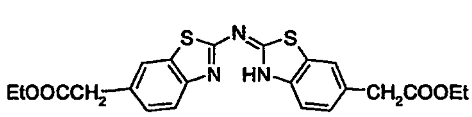,

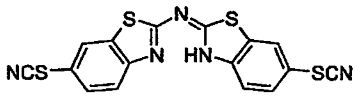, 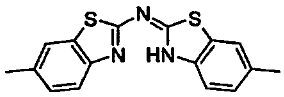,

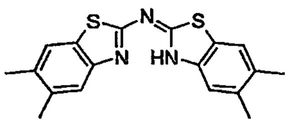, 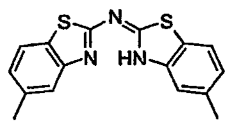, 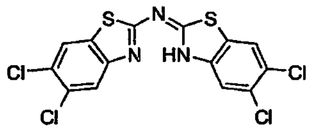,

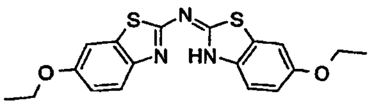, 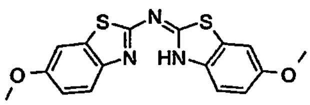,

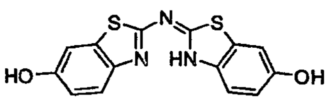, 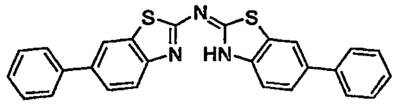,

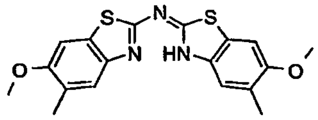, 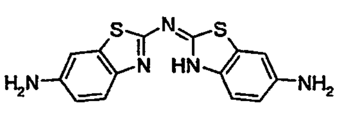,

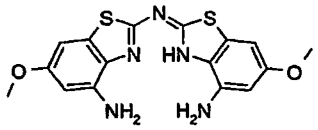, 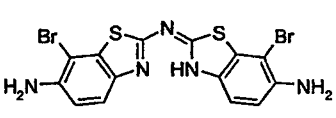,

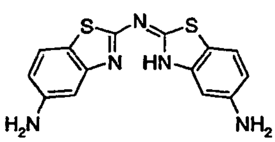, 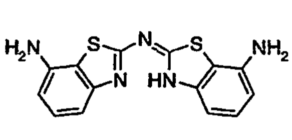, 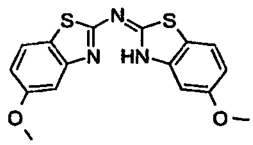,

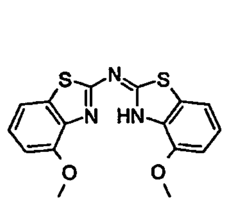, 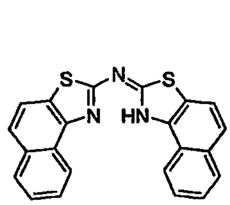, 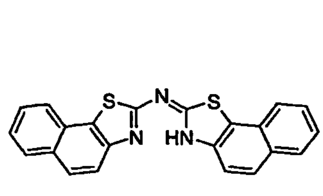,

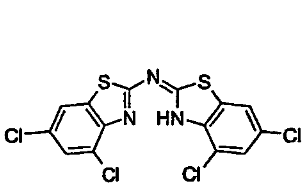, 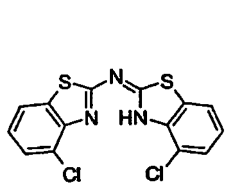, 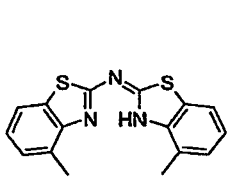,

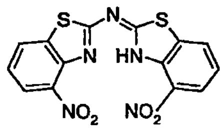,
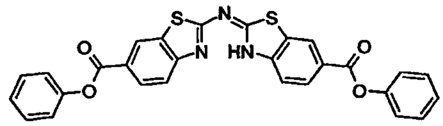,

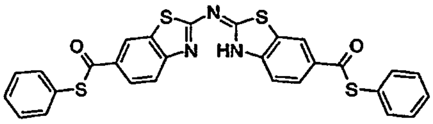,
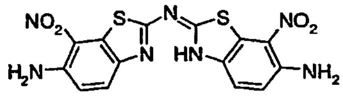,

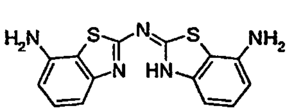,
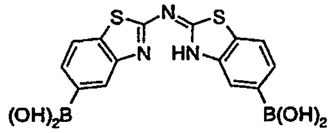,

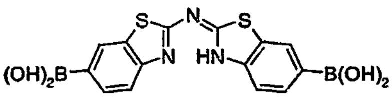,
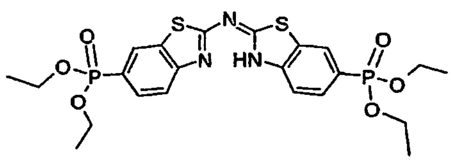,

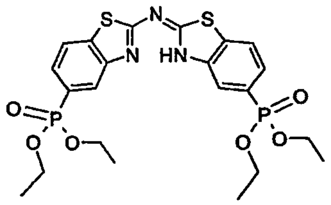,
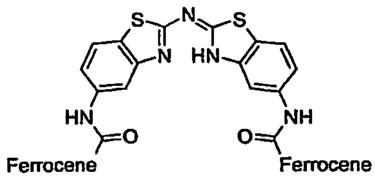,

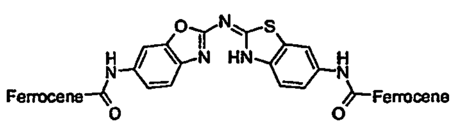,
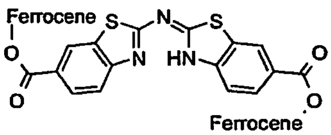,

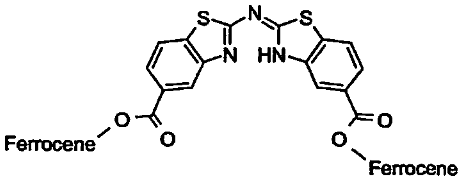,
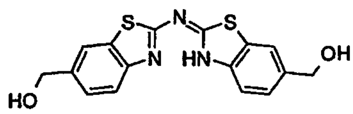,

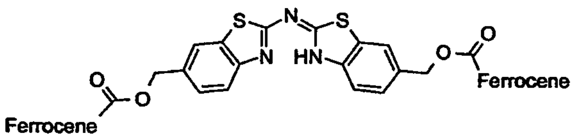,
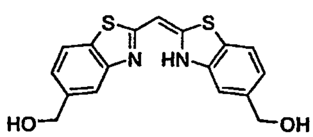,

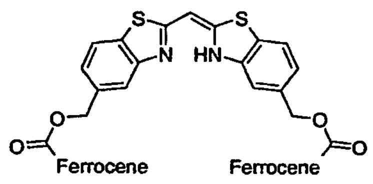 , 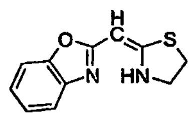 , 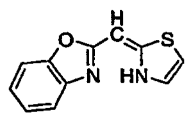 ,

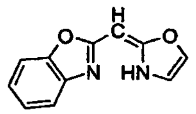 , 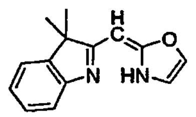 , 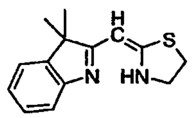 , 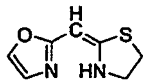 ,

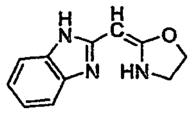 , 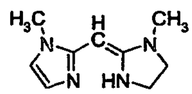 , 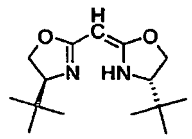 , 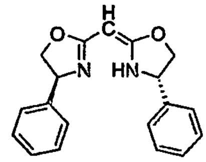 ,

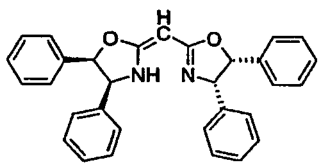 , 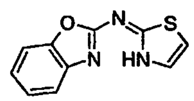 , 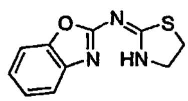 ,

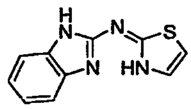 , 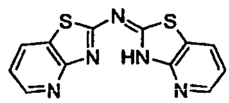 , 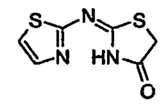 , 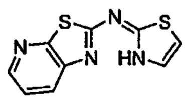 ,

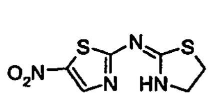 and 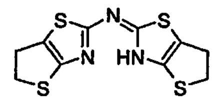 .

Especially interesting properties are exhibited by the preferred compounds of formulae

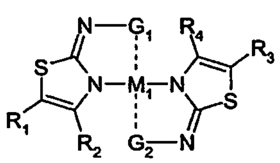 (II) and 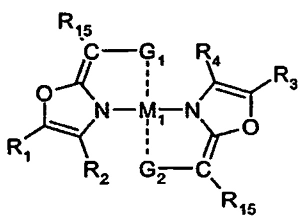 (III)

and also mixtures of compounds of formula (II) and/or (III), wherein especially $G_1$ and $G_2$ are the preferred heterocydes disclosed above and at the same time or independently thereof $M_1$ is a preferred transition metal. $A_3$ in $G_1$ and $G_2$ can be especially $N(R_{12})$, O, S or, especially in formula (III), $C(C_1\text{-}C_5alkyl)_2$ -

Special preference is given to compounds of formula

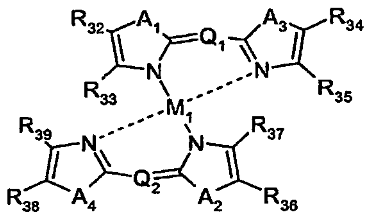
(IV)

and mixtures thereof wherein $A_4$, independently of $A_3$, has the same definition and the same preferred meanings as $A_3$.

Very special preference is given to compounds of formula

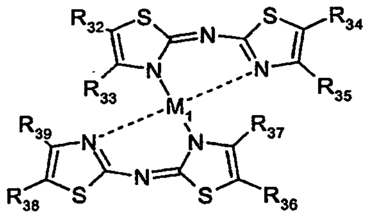
(V).

Both in formula (IV) and in formula (V), $R_{32}$, $R_{33}$, $R_{34}$, $R_{35}$, $R_{36}$, $R_{37}$, $R_{38}$ and $R_{39}$ are preferably H, $C_1$-$C_4$alkyl, COO-$C_1$-$C_4$alkyl, CN, $NO_2$, CHO, $COC_1$-$C_4$alkyl, phenyl, CH[-O-$C_2$-$C_3$alkylene-O-], C($C_1$-$C_4$alkyl)[-O-$C_2$-$C_3$alkylene-O-], $CH=C(CN)_2$, $C(CN)=C(CN)_2$ or $C(C_1$-$C_4alkyl)=C(CN)_2$, especially H, $CH_3$, $C_2H_5$, $COOCH_3$, $COOC_2H_5$, CN, $NO_2$ or CHO.

Compounds of formula (IV) are, for example, the following:

| N° | Formula | $A_1=A_2$ | $A_3=A_4$ | $Q_1=Q_2$ | $R_{32}=R_{36}$ | $R_{33}=R_{37}$ | $R_{34}=R_{38}$ | $R_{35}=R_{39}$ | $M_1$ |
|---|---|---|---|---|---|---|---|---|---|
| D1 | (IV) | $NCH_3$ | O | N | H | H | H | H | $Co^{2+}$ |
| D2 | (IV) | $NCH_2CH_3$ | O | N | $CH_3$ | H | $CH_3$ | H | $Co^{2+}$ |
| D3 | (IV) | $NCH_2CH_3$ | O | N | $CH_3$ | H | H | H | $Co^{2+}$ |
| D4 | (IV) | S | O | N | $NO_2$ | H | H | H | $Co^{2+}$ |
| D5 | (IV) | S O N | | | $NO_2$ | H | $CH_3$ | H | $Co^{2+}$ |
| D6 | (IV) | $NCH_3$ | $NCH_3$ | N | $NO_2$ | H | $NO_2$ | H | $Co^{2+}$ |
| D7 | (IV) | $NCH_2CH_3$ | $NCH_2CH_3$ | N | CN | H | CN | H | $Co^{2+}$ |
| D8 | (IV) | S | $NCH_2CH_3$ | N | H | H | H | H | $Co^{2+}$ |
| D9 | (IV) | O | O | CH | $NO_2$ | H | $NO_2$ | H | $Co^{2+}$ |
| D10 | (IV) | O | O | CH | CN | H | CN | H | $Co^{2+}$ |
| D11 | (IV) | O | O | CH | $SCH_3$ | H | $SCH_3$ | H | $Co^{2+}$ |
| D12 | (IV) | S | S | N | t-$C_4H_9$ | H | t-$C_4H_9$ | H | $Co^{2+}$ |
| D13 | (IV) | S | S | N | CHO | H | CHO | H | $Co^{2+}$ |
| D14 | (IV) | S | S | N | H | i-$C_3H_7$ | H | i-$C_3H_7$ | $Co^{2+}$ |
| D15 | (IV) | S | S | N | $CHC(CN)_2$ | H | $CHC(CN)_2$ | H | $Co^{2+}$ |
| D16 | (IV) | $NCH_3$ | O | N | H | H | H | H | $Cu^{2+}$ |
| D17 | (IV) | $NCH_2CH_3$ | O | N | $CH_3$ | H | $CH_3$ | H | $Cu^{2+}$ |
| D18 | (IV) | $NCH_2CH_3$ | O | N | $CH_3$ | H | H | H | $Ni^{2+}$ |
| D19 | (IV) | S | O | N | $NO_2$ | H | H | H | $Cu^{2+}$ |
| D20 | (IV) | S | O | N | $NO_2$ | H | $CH_3$ | H | $Cu^{2+}$ |
| D21 | (IV) | $NCH_3$ | $NCH_3$ | N | $NO_2$ | H | $NO_2$ | H | $Ni^{2+}$ |
| D22 | (IV) | $NCH_2CH_3$ | $NCH_2CH_3$ | N | CN | H | CN | H | $Cu^{2+}$ |
| D23 | (IV) | S | $NCH_2CH_3$ | N | H | H | H | H | $Cu^{2+}$ |
| D24 | (IV) | O | O | CH | $NO_2$ | H | $NO_2$ | H | $Ni^{2+}$ |
| D25 | (IV) | O | O | CH | CN | H | CN | H | $Cu^{2+}$ |
| D26 | (IV) | O | O | CH | $SCH_3$ | H | $SCH_3$ | H | $Cu^{2+}$ |

Table continued

| N° | Formula | $A_1$=$A_2$ | $A_3$=$A_4$ | $Q_1$=$Q_2$ | $R_{32}$=$R_{36}$ | $R_{33}$=$R_{37}$ | $R_{34}$=$R_{38}$ | $R_{35}$=$R_{39}$ | $M_1$ |
|---|---|---|---|---|---|---|---|---|---|
| D27 | (IV) | S | S | N | t-$C_4H_9$ | H | t-$C_4H_9$ | H | $Ni^{2+}$ |
| D28 | (IV) | S | S | N | CHO | H | CHO | H | $Cu^{2+}$ |
| D29 | (IV) | S | S | N | H | i-$C_3H_7$ | H | i-$C_3H_7$ | $Cu^{2+}$ |
| D30 | (IV) | S | S | N | CHC $(CN)_2$ | H | $CHC(CN)_2$ | H | $Ni^{2+}$ |

Compounds of formula (V) are, for example, the following:

| N° | Formula | $R_{32}$=$R_{36}$ | $R_{33}$=$R_{37}$ | $R_{34}$=$R_{38}$ | $R_{35}$=$R_{39}$ | $M_1$ |
|---|---|---|---|---|---|---|
| D31 | (V) | H | H | H | H | $Co^{2+}$ |
| D32 | (V) | $CH_3$ | H | $CH_3$ | H | $Co^{2+}$ |
| D33 | (V) | $CH_3$ | H | H | H | $Co^{2+}$ |
| D34 | (V) | H | $CH_3$ | H | $CH_3$ | $Co^{2+}$ |
| D35 | (V) | H | H | H | $CH_3$ | $Co^{2+}$ |
| D36 | (V) | $CH_2CH_3$ | H | $CH_2CH_3$ | H | $Co^{2+}$ |
| D37 | (V) | $CH_2CH_3$ | H | H | H | $Co^{2+}$ |
| D38 | (V) | n-$C_3H_7$ | H | n-$C_3H_7$ | H | $Co^{2+}$ |
| D39 | (V) | n-$C_3H_7$ | H | H | H | $Co^{2+}$ |
| D40 | (V) | i-$C_3H_7$ | H | i-$C_3H_7$ | H | $Co^{2+}$ |
| D41 | (V) | i-$C_3H_7$ | H | H | H | $Co^{2+}$ |
| D42 | (V) | n-$C_4H_9$ | H | n-$C_4H_9$ | H | $Co^{2+}$ |
| D43 | (V) | n-$C_4H_9$ | H | H | H | $Co^{2+}$ |
| D44 | (V) | i-$C_3H_7$ | $CH_3$ | i-$C_3H_7$ | $CH_3$ | $Co^{2+}$ |
| D45 | (V) | H | H | i-$C_3H_7$ | $CH_3$ | $Co^{2+}$ |
| D46 | (V) | H | i-$C_4H_9$ | H | i-$C_4H_9$ | $Co^{2+}$ |
| D47 | (V) | H | i-$C_4H_9$ | H | H | $Co^{2+}$ |
| D48 | (V) | $COOCH_2CH_3$ | $CH_3$ | $COOCH_2CH_3$ | $CH_3$ | $Co^{2+}$ |
| D49 | (V) | $COOCH_2CH_3$ | $CH_3$ | H | H | $Co^{2+}$ |
| D50 | (V) | H | $COOCH_2CH_3$ | H | H | $Co^{2+}$ |
| D51 | (V) | H | $COOCH_2CH_3$ | H | $COOCH_2CH_3$ | $Co^{2+}$ |
| D52 | (V) | $CH=C(CN)_2$ | H | $CH=C(CN)_2$ | H | $Co^{2+}$ |
| D53 | (V) | $NO_2$ | H | $NO_2$ | H | $Co^{2+}$ |
| D54 | (V) | H | H | H | H | $Cu^{2+}$ |
| D55 | (V) | $CH_3$ | H | $CH_3$ | H | $Cu2^{+}$ |
| D56 | (V) | $CH_3$ | H | H | H | $Cu^{2+}$ |
| D57 | (V) | H | $CH_3$ | H | $CH_3$ | $Cu^{2+}$ |
| D58 | (V) | H | H | H | $CH_3$ | $Cu^{2+}$ |
| D59 | (V) | $CH_2CH_3$ | H | $CH_2CH_3$ | H | $Cu^{2+}$ |
| D60 | (V) | $CH_2CH_3$ | H | H | H | $Cu^{2+}$ |
| D61 | (V) | n-$C_3H_7$ | H | n-$C_3H_7$ | H | $Cu^{2+}$ |
| D62 | (V) | n-$C_3H_7$ | H | H | H | $Cu^{2+}$ |
| D63 | (V) | i-$C_3H_7$ | H | i-$C_3H_7$ | H | $Cu^{2+}$ |
| D64 | (V) | i-$C_3H_7$ | H | H | H | $Cu^{2+}$ |
| D65 | (V) | n-$C_4H_9$ | H | n-$C_4H_9$ | H | $Cu^{2+}$ |
| D66 | (V) | n-$C_4H_9$ | H | H | H | $Cu^{2+}$ |
| D67 | (V) | i-$C_3H_7$ | $CH_3$ | i-$C_3H_7$ | $CH_3$ | $Cu^{2+}$ |
| D68 | (V) | H | H | i-$C_3H_7$ | $CH_3$ | $Cu^{2+}$ |
| D69 | (V) | H | i-$C_4H_9$ | H | i-$C_4H_9$ | $Cu^{2+}$ |
| D70 | (V) | H | i-$C_4H_9$ | H | H | $Cu^{2+}$ |
| D71 | (V) | $COOCH_2CH_3$ | $CH_3$ | $COOCH_2CH_3$ | $CH_3$ | $Cu^{2+}$ |

Table continued

| N° | Formula | $R_{32}=R_{36}$ | $R_{33}=R_{37}$ | $R_{34}=R_{38}$ | $R_{35}=R_{39}$ | $M_1$ |
|---|---|---|---|---|---|---|
| D72 | (V) | $COOCH_2CH_3$ | $CH_3$ | H | H | $Cu^{2+}$ |
| D73 | (V) | H | $COOCH_2CH_3$ | H | H | $Cu^{2+}$ |
| D74 | (V) | H | $COOCH_2CH_3$ | H | $COOCH_2CH_3$ | $Cu^{2+}$ |
| D75 | (V) | $CH=C(CN)_2$ | H | $CH=C(CN)_2$ | H | $Cu^{2+}$ |
| D76 | (V) | $NO_2$ | H | $NO_2$ | H | $Cu^{2+}$ |
| D77 | (V) | H | H | H | H | $Ni^{2+}$ |
| D78 | (V) | $CH_3$ | H | $CH_3$ | H | $Ni^{2+}$ |
| D79 | (V) | $CH_3$ | H | H | H | $Ni^{2+}$ |
| D80 | (V) | H | $CH_3$ | H | $CH_3$ | $Ni^{2+}$ |
| D81 | (V) | H | H | H | $CH_3$ | $Ni^{2+}$ |
| D82 | (V) | $CH_2CH_3$ | H | $CH_2CH_3$ | H | $Ni^{2+}$ |
| D83 | (V) | $CH_2CH_3$ | H | H | H | $Ni^{2+}$ |
| D84 | (V) | $n-C_3H_7$ | H | $n-C_3H_7$ | H | $Ni^{2+}$ |
| D85 | (V) | $n-C_3H_7$ | H | H | H | $Ni^{2+}$ |
| D86 | (V) | $i-C_3H_7$ | H | $i-C_3H_7$ | H | $Ni^{2+}$ |
| D87 | (V) | $i-C_3H_7$ | H | H | H | $Ni^{2+}$ |
| D88 | (V) | $n-C_4H_9$ | H | $n-C_4H_9$ | H | $Ni^{2+}$ |
| D89 | (V) | $n-C_4H_9$ | H | H | H | $Ni^{2+}$ |
| D90 | (V) | $i-C_3H_7$ | $CH_3$ | $i-C_3H_7$ | $CH_3$ | $Ni^{2+}$ |
| D91 | (V) | H | H | $i-C_3H_7$ | $CH_3$ | $Ni^{2+}$ |
| D92 | (V) | H | $i-C_4H_9$ | H | $i-C_4H_9$ | $Ni^{2+}$ |
| D93 | (V) | H | $i-C_4H_9$ | H | H | $Ni^{2+}$ |
| D94 | (V) | $COOCH_2CH_3$ | $CH_3$ | $COOCH_2CH_3$ | $CH_3$ | $Ni^{2+}$ |
| D95 | (V) | $COOCH_2CH_3$ | $CH_3$ | H | H | $Ni^{2+}$ |
| D96 | (V) | H | $COOCH_2CH_3$ | H | H | $Ni^{2+}$ |
| D97 | (V) | H | $COOCH_2CH_3$ | H | $COOCH_2CH_3$ | $Ni^{2+}$ |
| D98 | (V) | $CH=C(CN)_2$ | H | $CH=C(CN)_2$ | H | $Ni^{2+}$ |
| D99 | (V) | $NO_2$ | H | $NO_2$ | H | $Ni^{2+}$ |

Instead of pure compounds it is also possible to use mixtures thereof, for example the following mixtures:

| N° | | % | | % | | % | | % | | % |
|---|---|---|---|---|---|---|---|---|---|---|
| M1 | D54 | 80 | D77 | 20 | | | | | | |
| M2 | D32 | 5 | D54 | 80 | D77 | 15 | | | | |
| M3 | D54 | 80 | D55 | 10 | D78 | 10 | | | | |
| M4 | D54 | 80 | D56 | 15 | D79 | 5 | | | | |
| M5 | D54 | 80 | D55 | 10 | D56 | 10 | | | | |
| M6 | D54 | 80 | D56 | 20 | | | | | | |
| M7 | D54 | 80 | D56 | 15 | D79 | 5 | | | | |
| M8 | D54 | 90 | D57 | 10 | | | | | | |
| M9 | D54 | 80 | D57 | 10 | D81 | 10 | | | | |
| M10 | D54 | 80 | D58 | 20 | | | | | | |
| M11 | D54 | 80 | D57 | 10 | D58 | 10 | | | | |
| M12 | D54 | 65 | D57 | 10 | D58 | 5 | D77 | 15 | D81 | 5 |
| M13 | D54 | 90 | D59 | 10 | | | | | | |
| M14 | D54 | 80 | D60 | 20 | | | | | | |
| M15 | D54 | 70 | D59 | 5 | D60 | 10 | D76 | 5 | D82 | 5 |
| M16 | D56 | 90 | D61 | 10 | | | | | | |
| M17 | D56 | 90 | D62 | 10 | | | | | | |
| M18 | D54 | 90 | D68 | 10 | | | | | | |

Table continued

| N° | | % | | % | | % | | % | | % |
|---|---|---|---|---|---|---|---|---|---|---|
| M19 | D54 | 90 | D71 | 10 | | | | | | |
| M20 | D54 | 75 | D72 | 15 | D76 | 5 | D95 | 5 | | |
| M21 | D54 | 70 | D73 | 20 | D76 | 5 | D96 | 5 | | |
| M22 | D54 | 80 | D74 | 20 | | | | | | |
| M23 | D54 | 80 | D55 | 5 | D56 | 5 | D57 | 5 | D76 | 5 |
| M24 | M5 | 50 | M14 | 30 | D58 | 5 | D76 | 5 | D77 | 10 |

Instead of preparing mixtures by mixing together the components, it is favourably possible to prepare mixtures by mixed synthesis, the metals being added in any desired order in succession or preferably simultaneously to a pre-prepared mixture of the ligands, or conversely the ligands being added in any desired order in succession or preferably all of them simultaneously to a pre-prepared mixture of the metals. The mixtures prepared by mixed synthesis generally have somewhat better solubility than physical mixtures, possibly because of their asymmetric components.

In addition to comprising one or more compounds of formula (I) and optionally customary additives, the optical recording media according to the invention may also comprise other chromophores, preferably metal-free chromophores. Other chromophores may, if desired, be added in an amount of from 1 to 200 % by weight, based on the total of the compounds of formula (I). The amount of other chromophores is preferably from 5 to 100 % by weight, especially from 10 to 50 % by weight, based on the total of the compounds of formula (I). Chromophores can be dyes or UV absorbers, preferably having an absorption maximum of from 350 to 400 nm or at from 600 to 700 nm, for example around 380 or 630 nm.

Especially preferred additional metal-free chromophores are cyanines, azacyanines, merocyanines and oxonols and also rhodamines, for example those disclosed in WO 04/006878, WO 02/082438 or EP-A-1 083 555, and also

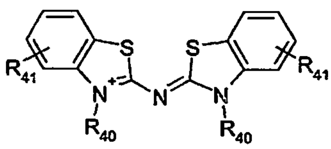, 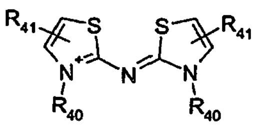, 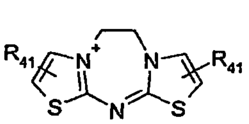,

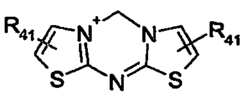, 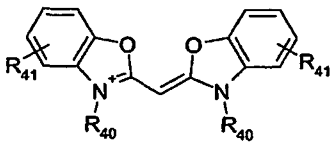, 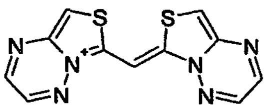,

wherein $R_{40}$ is $C_1$-$C_{24}$alkyl or $C_2$-$C_{24}$alkenyl, each of which can be unsubstituted or substituted, and $R_{41}$ is any substituent $R_{40}$ may be, for example, methyl, ethyl, vinyl, allyl, isopropyl, n-butyl, 2-isopropyloxy-ethyl, n-pentyl, 3-methyl-butyl, 3,3-dimethylbutyl, 2-ethyl-hexyl, 2-cyano-ethyl, furan-2-yl-methyl or 2-hydroxy-methyl; $R_{41}$ is, for example, $C_6$-$C_{10}$aryl, $C_1$-$C_{24}$alkyl or $C_2$-$C_{24}$alkenyl.
Purely illustrative examples of such chromophores are:

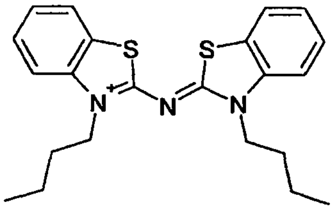, 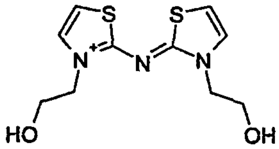,

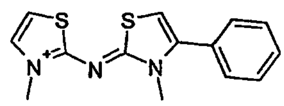 , 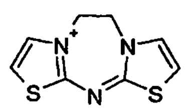 , 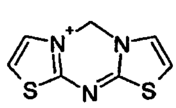 ,

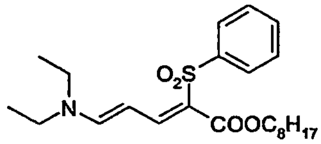 , 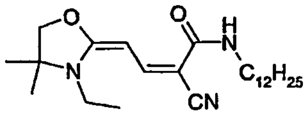 ,

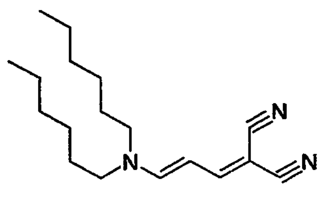 , 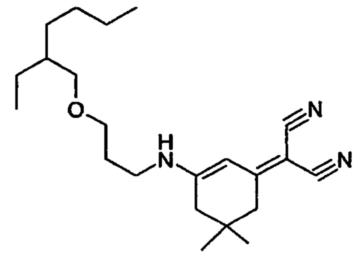 ,

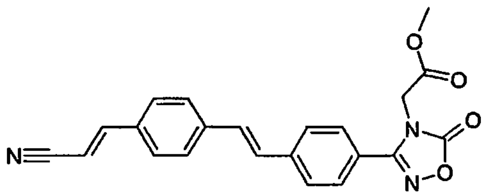 , 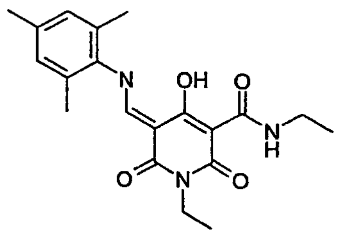 ,

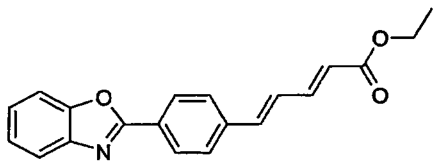 , 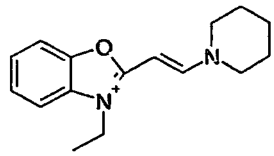 ,

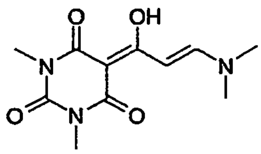 , 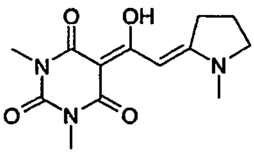 and 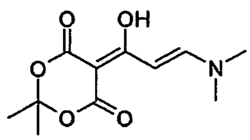 .

The following Examples illustrate the invention but do not limit the scope thereof (unless otherwise indicated,"%" always refers to % by weight):

Example 1: 1.0 g of the compound of formula

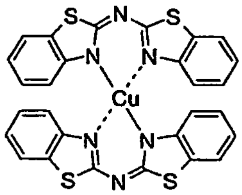

is applied in the form of a dichloromethane solution to glass. The solid layer is irradiated for 90 hours with xenon light according to ISO-105-B02 (Atlas Ci-35 Weather-O-meter, 15 kJ/$cm^2$). The light stability is excellent (see Example 7).

Example 2: On the solid layer according to Example 1, marks are written into the recording layer using a pulsed dye laser (15 ns pulse length) at a wavelength of 405 nm at an energy density of 0.8 kJ/$m^2$. The written sites exhibit a resulting change in reflectivity.

Example 3: 0.5 g of the compound according to Example 1 is dissolved in 99.5 g of dioxane and applied by means of spin-coating to a silicon wafer. The colourless solid layer is measured using a spectral ellipsometer (Sopra). At a wavelength of 405 nm a refractive index of 2.52 is determined.

Example 4: 1.0 g of the compound of formula

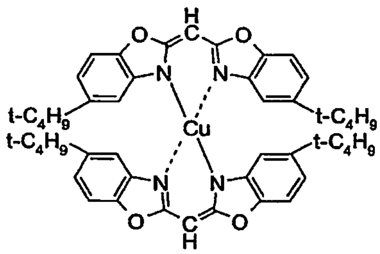

is dissolved in 99 g of methylcyclohexane and filtered through a 0.2 μm Teflon filter. The dye solution is then applied by rotation at 250 rev/min to a 1.2 mm thick, flat polycarbonate plate (diameter 120 mm). The rotational speed is then increased to 1200 rev/min, so that the excess solution is spun off, and a uniform solid layer is formed. After drying, the solid layer has an absorption of 0.61 at 382 nm. Using an optical measuring system (ETA-RT, STEAG ETA-Optik), the layer thickness and the complex refractive index are determined. At 405 nm the dye layer has a layer thickness of 56 nm, a refractive index n of 1.95 and an extinction coefficient k of 0.090.

Example 5:

Synthesis of the ligand

19.4 ml of a 55 % aqueous chloracetaldehyde solution are added to a suspension of 10.2 g of dithiobiuret in 45 ml of ethanol. The reaction mixture is heated at 75°C for 2 hours, and then poured into 150 ml of water. After the addition of 200 ml of an aqueous sodium acetate solution (4.6N), the precipitate is filtered off, washed with water and dried at 50°C/1.5· $10^3$ Pa, yielding 8.8 g of crude product, and after recrystallisation from ethanol 6.7 g of pure product of formula

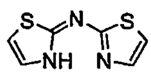

(m.p. 212°C).

Synthesis of the complex

0.5N aqueous copper(II) acetate solution is added to a solution of 458 mg of the resulting ligand in 30 ml of ethanol until precipitation, which begins immediately, is complete. After filtration, the product is washed with ethanol and diethyl ether, and then dried at 70°C/1.5· $10^3$ Pa. 390 mg of pure product of formula

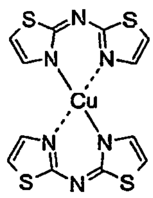

(decomp. 264°C) are obtained.

Example 6: 1.0 g of the complex according to Example 5 is dissolved in 99 g of 2,2,3,3-tetrafluoro-1-propanol and filtered through a 0.2 μm Teflon filter. The dye solution is then applied by rotation at 250 rev/min to a 1.2 mm thick, flat polycarbonate plate (diameter 120 mm); the rotational speed is then increased to 1500 rev/min, so that the excess solution is spun off and a uniform solid layer is formed. After drying, the solid layer has an absorption of 0.35 at 356 nm. Using an optical measuring system (ETA-RT, STEAG ETA-Optik), the layer thickness and the complex refractive index are determined. At 405 nm the dye layer has a layer thickness of 18 nm, a refractive index n of 2.25 and an extinction coefficient k of 0.031.

Examples 7 - 38: The procedure is analogous to Example 6, but instead of the complex of Example 5 the following ligands and metal cations are used:

| Metal | Ligand(2×) | $\lambda_{max}$ [nm] | Solvent | n | k |
|---|---|---|---|---|---|
| $Cu^{2+}$ | 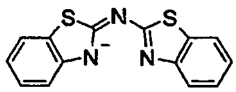 | 370 | $CH_3CN$ (+HCl) | 2.52 | 0.47 |
| $Cu^{2+}$ | 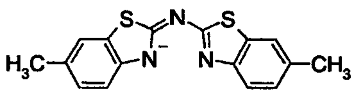 | 383 | $CH_2Cl_2$ | 2.30 | 0.185 |
| $Ni^{2+}$ | 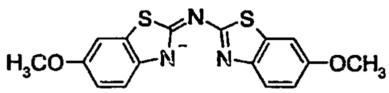 | 370 | $CH_2Cl_2$, | 2.23 | 0.160 |
| $Cu^{2+}$ | 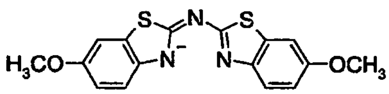 | 390 | $CH_2Cl_2$ | 2.22 | 0.266 |
| $Cu^{2+}$ | 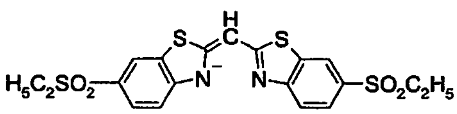 | 377 | $CH_2Cl_2$ | 2.21 | 0.244 |
| $Co^{2}+$ | 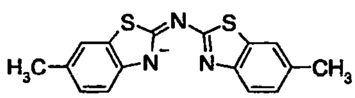 | 370 | $CH_2Cl_2$ | 2.20 | 0.137 |
| $Co^{2+}$ | 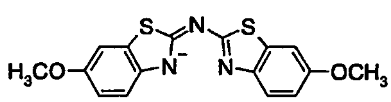 | 376 | $CH_2Cl_2$ | 2.19 | 0.179 |
| $Ni^{2+}$ | 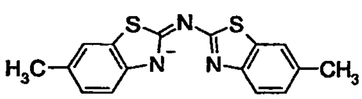 | 361 | $CH_2Cl_2$ | 2.17 | 0.108 |

Table continued

| Metal | Ligand(2×) | $\lambda_{max}$ [nm] | Solvent | n | k |
|---|---|---|---|---|---|
| $Co^{2+}$ | 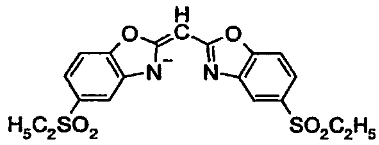 | 370 | $CH_2Cl_2$ | 2.08 | 0.158 |
| $Ni^{2+}$ | 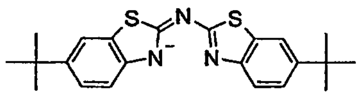 | 361 | $CH_2Cl_2$ | 2.07 | 0.097 |
| $Cu^{2+}$ | 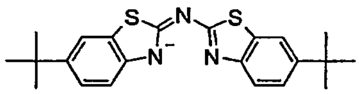 | 384 | $CH_2Cl_2$ | 2.00 | 0.092 |
| $Co^{2+}$ | 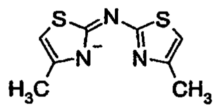 | 351 | $CH_2Cl_2$ | 1.98 | 0.100 |
| $Co^{2+}$ | 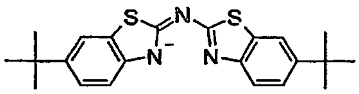 | 370 | $CH_2Cl_2$ | 1.94 | 0.083 |
| $Ni^{2}+$ | 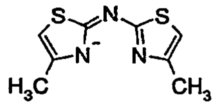 | 350 | $CH_2Cl_2$ | 1.93 | 0.083 |
| $Co^{2+}$ | 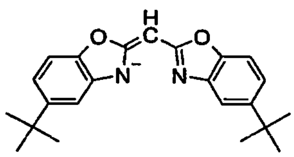 | 367 | $CH_2Cl_2$ | 1.83 | 0.103 |
| $Ni^{2+}$ | 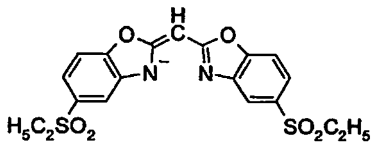 | 368 | $CH_2Cl_2$ | 1.74 | 0.119 |
| $Cu^{2+}$ | 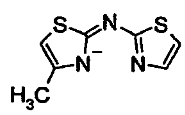 | 369 | $CH_2Cl_2$ | | |
| $Ni^{2+}$ | 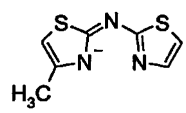 | 350 | $CH_2Cl_2$ | | |
| $Co^{2+}$ | 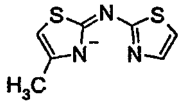 | 348 | $CH_2Cl_2$ | | |

Table continued

| Metal | Ligand(2×) | $\lambda_{max}$ [nm] | Solvent | n | k |
|---|---|---|---|---|---|
| $Ni^{2+}$ | 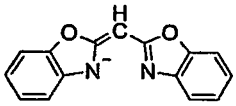 | 356 | $CH_3CN$ | | |
| $Ni^{2+}$ | 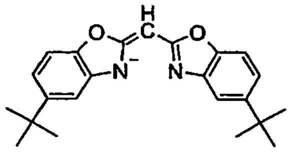 | 357 | $CH_2Cl_2$ | | |
| $Co^{2+}$ | 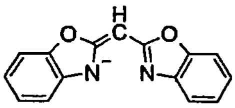 | 364 | $CH_2Cl_2$ | | |
| $Co^{2+}$ | 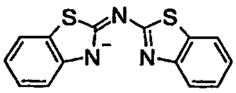 | 370 | $CH_3CN$ (+HCl) | | |
| $Ni^{2+}$ | 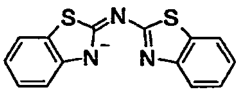 | 370 | $CH_3CN$ (+HCl) | | |
| $Cu^{2+}$ | 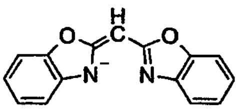 | 374 | $CH_2Cl_2$ | | |
| $Ni^{2+}$ | 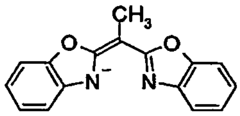 | 374 | $CH_2Cl_2$ | | |
| $Co^{2+}$ | 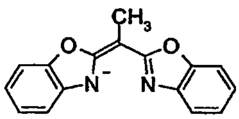 | 376 | $CH_3CN$ | | |
| $Cu^{2+}$ | 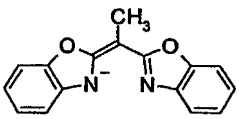 | 387 | $CH_2Cl_2$ | | |
| $Cu^{2+}$ | 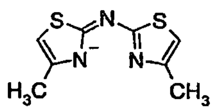 | | | | |
| $Ni^{2+}$ | 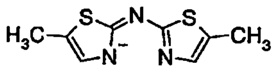 | | | | |

Table continued

| Metal | Ligand(2×) | $\lambda_{max}$ [nm] | Solvent | n | k |
|---|---|---|---|---|---|
| $Co^{2+}$ | 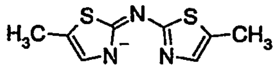 | 350 | $CH_2Cl_2$ | | |
| $Cu^{2+}$ | 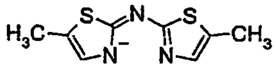 | 358 | $CH_2Cl_2$ | | |

Examples 39 - 62: The procedure is analogous to Example 6, but instead of the complex of Example 5 the mixtures M1 to M24 are used.

Examples 63 - 86: The procedure is analogous to Example 6, but instead of the complex of Example 5 there are used mixtures having the same proportions of metal and ligand as mixtures M1 to M24 but the complexes are prepared by mixed synthesis (variant of the simultaneous addition of the metal mixture to the ligand mixture). The results are similar to those of Examples 39 - 62, but have surprisingly better solubility and solution stability.

Examples 87 - 94: The procedure is analogous to Example 6, but instead of the complex of Example 5 there are used the following mixtures of compounds of formula (I) with cyanines and merocyanines:

80% 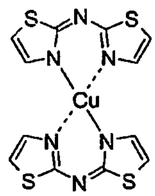 + 20% 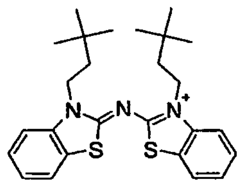 ;

80% 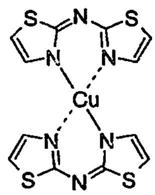 + 20% 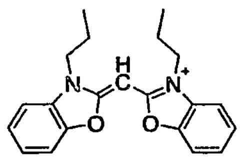 ;

75% 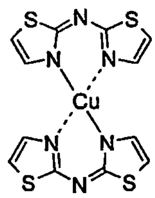 + 25% 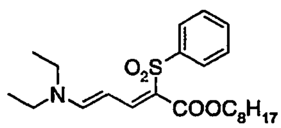 ;

75% 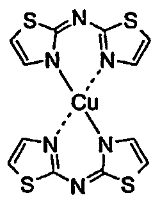 + 25% 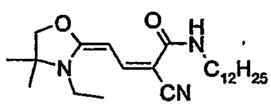 ;

75% 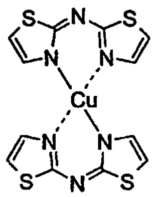 + 25% 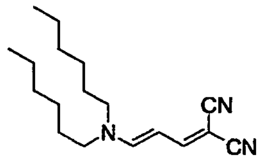 ;

75% 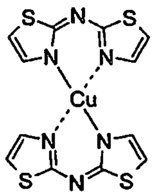 + 25% 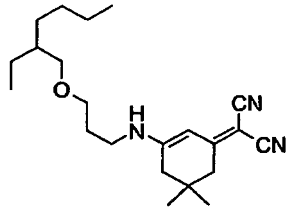 ;

50% 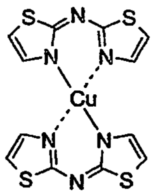 + 50% 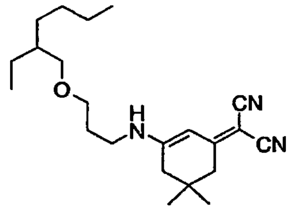 ;

95% 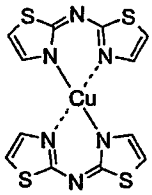 + 5% 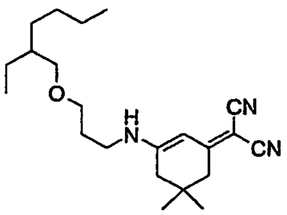 .

It will be understood that it is also possible to combine mixtures of those cyanines, merocyanines and/or also other chromophores with complexes of formula (I) or with mixtures of complexes of formula (I), the results achieved generally being very good.

## Claims

1. An optical recording medium comprising a substrate, a recording layer and optionally one or more reflecting layers, wherein the recording layer comprises a compound of formula

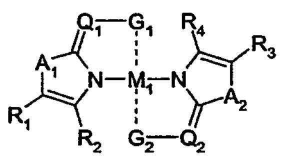 (I)

or a tautomer thereof, wherein

$G_1$ and $G_2$ are each independently of the other

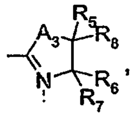, 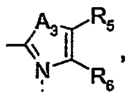, 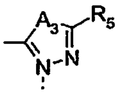 or 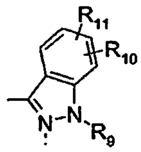,

$A_1$ and $A_2$ are each independently of the other $N(R_{12})$, O, S or Se and $A_3$ is $C(C_1\text{-}C_5alkyl)_2$, $C(C_4\text{-}C_5alkylene)$, N $(R_{12})$, O, S, Se, $N{=}C(R_{13})$ or unsubstituted or $R_{14}$-substituted CH=CH;

$M_1$ is a transition metal of groups IX to XII, preferably Co, Cu, Ni, Pd or Zn, especially Co, Cu or Ni;

$Q_1$ and $Q_2$ are each independently of the other $C(R_{15})$, N or P;

$R_1$, $R_2$, $R_3$, $R_4$, $R_5$, $R_6$, $R_7$, $R_8$ and $R_{14}$ are each independently of the others hydrogen, $R_{18}$, or $C_6$-$C_{12}$aryl, $C_4$-$C_{12}$heteroaryl, $C_7$-$C_{12}$aralkyl or $C_5$-$C_{12}$heteroaralkyl each unsubstituted or substituted by one or more, where applicable identical or different, radicals $R_{18}$; or

$R_1$ and $R_2$, $R_3$ and $R_4$, $R_5$ and $R_6$, $R_5$ and $R_{13}$ and/or $R_5$ and $R_{14}$, together in pairs, are $C_3$-$C_6$alkylene or $C_3$-$C_6$alkenylene, each of which is unsubstituted or substituted by one or more, where applicable identical or different, radicals $R_{17}$ and may be uninterrupted or interrupted by O, S or $N(R_{12})$, or 1,4-buta-1,3-dienylene,

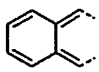

or

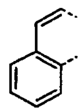,

each of which is unsubstituted or substituted by one or more, where applicable identical or different, radicals $R_{18}$ and in which 1 or 2 carbon atoms may have been replaced by nitrogen;

$R_9$, $R_{12}$ and $R_{13}$ are each independently of the others $C_1$-$C_{24}$alkyl, $C_3$-$C_{24}$cycloalkyl, $C_2$-$C_{24}$alkenyl, $C_3$-$C_{24}$cycloalkenyl, $C_1$-$C_4$alkyl-$[O\text{-}C_1\text{-}C_4alkylene]_m$ or $C_1$-$C_4$alkyl-$[NH\text{-}C_1\text{-}C_4alkylene]_m$ each of which is unsubstituted or substituted by one or more, where applicable identical or different, radicals $R_{17}$; or $C_6$-$C_{12}$aryl, $C_4$-$C_{12}$heteroaryl, $C_7$-$C_{12}$aralkyl or $C_5$-$C_{12}$heteroaralkyl, each of which is unsubstituted or substituted by one or more, where applicable identical or different, radicals $R_{18}$;

$R_{10}$, $R_{11}$ and $R_{18}$ are each independently of the others halogen, nitro, cyano, thiocyanato, hydroxy, O-$R_{19}$, O-CO-$R_{19}$, S-$R_{19}$, CHO, $COR_{20}$, $CHOR_{19}OR_{23}$, $CR_{20}OR_{19}OR_{23}$, $R_{16}$, N=N-$R_{16}$, $N{=}CR_{19}R_{20}$, $N{=}CR_{21}R_{22}$, $C(R_{15}){=}NR_{19}$, $C(R_{15}){=}NR_{21}$, $C(R_{15}){=}CR_{21}R_{22}$, $NH_2$, NH-$R_{19}$, $NR_{19}R_{20}$, $NH_3^+$, $NH_2R_{19}$+, $NHR_{19}R_{20}^+$, $NR_{19}R_{20}R_{23}^+$, $CONH_2$, $CONHR_{19}$, $CONR_{19}R_{20}$, $SO_2R_{19}$, $SO_2NH_2$, $SO_2NHR_{19}$, $SO_2NR_{19}R_{20}$, COOH, $COOR_{19}$, $OCOOR_{19}$, $NHCOR_{19}$, $NR_{19}COR_{23}$, $NHCOOR_{19}$, $NR_{19}COOR_{23}$, ureido, $NR_{19}$-CO-$NHR_{23}$, $B(OH)_2$, $B(OH)(OR_{19})$, $B(OR_{19})OR_{23}$, phosphato, $PR_{19}R_{23}$, $POR_{19}OR_{23}$, $P(=O)OR_{19}OR_{23}$, $OPR_{19}R_{23}$, $OPR_{19}OR_{23}$, $OP(=O)R_{19}OR_{23}$, $OP(=O)OR_{19}OR_{23}$, $OPO_3R_{19}$, sulfato, sulfo, or $C_1$-$C_{12}$alkyl, $C_3$-$C_{12}$cycloalkyl, $C_1$-$C_{12}$alkylthio, $C_3$-$C_{12}$cycloalkylthio, $C_1$-$C_{12}$alkoxy or $C_3$-$C_{12}$cycloalkoxy each unsubstituted or substituted by one or more, where applicable identical or different, radicals $R_{17}$;

$R_{15}$ is hydrogen, cyano, hydroxy, $C_1$-$C_{12}$alkoxy, $C_3$-$C_{12}$cycloalkoxy, $C_1$-$C_{12}$alkylthio, $C_3$-$C_{12}$cycloalkylthio, amino, $NHR_{24}$, $NR_{25}R_{26}$, $R_{27}$, halogen, nitro, formyl, N=N-$R_{27}$, $C(R_{14}){=}CR_{21}R_{22}$, $C(R_{14}){=}NR_{19}$, COO-$R_{25}$, carboxy, carbamoyl, CONH-$R_{25}$,

$CONR_{25}R_{26}$, $N{=}CR_{19}R_{20}$, or $C_1$-$C_{12}$alkyl, $C_3$-$C_{12}$cycloalkyl, $C_2$-$C_{12}$alkenyl or $C_3$-$C_{12}$cycloalkenyl each unsubstituted or substituted by one or more, where applicable identical or different, halogen, hydroxy, $C_1$-$C_{12}$alkoxy or $C_3$-$C_{12}$cycloalkoxy radicals;

$R_{16}$ is $C_6$-$C_{12}$aryl, $C_4$-$C_{12}$heteroaryl, $C_7$-$C_{12}$aralkyl or $C_5$-$C_{12}$heteroaralkyl, each of which is unsubstituted or substituted by one or more, where applicable identical or different, radicals $R_{26}$;

$R_{17}$ is halogen, hydroxy, O-$R_{25}$, O-CO-$R_{25}$, S-$R_{25}$, $NH_2$, NH-$R_{25}$, $NR_{25}R_{26}$, $NH_3^+$, $NH_2R_{25}^+$, $NHR_{25}R_{26}^+$, $NR_{24}R_{25}R_{26}^+$, $NR_{25}$-CO-$R_{24}$, $NR_{25}COOR_{24}$, cyano, formyl, COO-$R_{25}$, carboxy, carbamoyl, CONH-$R_{25}$, $CaNR_{25}R_{26}$, ureido, NH-CO-$NHR_{24}$, $NR_{25}$-CO-$NHR_{24}$, phosphato, $PR_{25}R_{24}$, $POR_{25}OR_{24}$, P(=O)$OR_{25}OR_{24}$, $OPR_{25}R_{24}$, $OPR_{25}OR_{24}$ , OP(=O)$R_{25}OR_{24}$, $OPO_3R_{25}$, OP(=O)$OR_{25}OR_{24}$, $SO_2R_{25}$, sulfato, sulfo, $R_{27}$, N=N-$R_{27}$, or $C_2$-$C_{12}$alkoxy or $C_1$-$C_{12}$cycloalkoxy each unsubstituted or mono- or poly-substituted by halogen;

$R_{19}$, $R_{20}$ and $R_{23}$ are each independently of the others $R_{16}$, or $C_1$-$C_{12}$alkyl, $C_3$-$C_{12}$cycloalkyl, $C_2$-$C_{12}$alkenyl or $C_3$-$C_{12}$cycloalkenyl, each of which is unsubstituted or substituted by one or more, where applicable identical or different, halogen, hydroxy, $C_1$-$C_{12}$alkoxy or $C_3$-$C_{12}$cycloalkoxy radicals; or

$R_{14}$ and $R_{19}$ together, $R_{15}$ and $R_{19}$ together and/or $R_{19}$ and $R_{23}$ together are $C_2$-$C_{12}$alkylene, $C_3$-$C_{12}$cycloalkylene, $C_2$-$C_{12}$alkenylene or $C_3$-$C_{12}$cycloalkenylene, each of which is unsubstituted or substituted by one or more, where applicable identical or different, halogen, hydroxy, $C_1$-$C_{12}$alkoxy or $C_3$-$C_{12}$cycloalkoxy radicals; or

$R_{19}$ and $R_{20}$ together with the common nitrogen are pyrrolidine, piperidine, piperazine or morpholine, each of which is unsubstituted or mono-to tetra-substituted by $C_1$-$C_4$alkyl; or carbazole, phenoxazine or phenothiazine, each of which is unsubstituted or substituted by one or more, where applicable identical or different, radicals $R_{28}$;

$R_{21}$ and $R_{22}$ are each independently of the other $NR_{25}R_{26}$, CN, $CONH_2$, $CaNHR_{19}$, $CONR_{19}R_{20}$ or $COOR_{20}$;

$R_{24}$, $R_{25}$ and $R_{26}$ are each independently of the others $C_1$-$C_{12}$alkyl, $C_3$-$C_{12}$cycloalkyl, $C_2$-$C_{12}$alkenyl, $C_3$-$C_{12}$cycloalkenyl, $C_6$-$C_{12}$aryl, $C_4$-$C_{12}$heteroaryl, $C_7$-$C_{12}$aralkyl or $C_5$-$C_{12}$heteroaralkyl; or

$R_{25}$ and $R_{26}$ together with the common nitrogen are pyrrolidine, piperidine, piperazine or morpholine, each of which is unsubstituted or mono- to tetra-substituted by $C_1$-$C_4$alkyl;

$R_{27}$ is $C_6$-$C_{12}$aryl, $C_4$-$C_{12}$heteroaryl, $C_7$-$C_{12}$aralkyl or $C_5$-$C_{12}$heteroaralkyl, each of which is unsubstituted or substituted by one or more, where applicable identical or different, radicals $R_{18}$;

$R_{28}$ is nitro, $SO_2NHR_{25}$. $SO_2NR_{25}R_{26}$, or $C_1$-$C_{12}$alkyl, $C_3$-$C_{12}$cycloalkyl, $C_1$-$C_{12}$alkylthio, $C_3$-$C_{12}$cycloalkylthio, $C_1$-$C_{12}$alkoxy or $C_3$-$C_{12}$cycloalkoxy, each of which is unsubstituted or substituted by one or more, where applicable identical or different, radicals $R_{17}$; and

m is a number from 1 to 10.

**2.** An optical recording medium according to claim 1, wherein $Q_1$ and $Q_2$ are C($R_{15}$);
$G_1$ and $G_2$ are

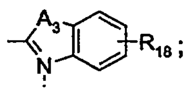

and $A_1$, $A_2$ and $A_3$ are O, S or N($R_{12}$);

$R_{12}$ is $C_1$-$C_{24}$alkyl, $C_1$-$C_4$alkyl-[O-$C_1$-$C_4$alkylene]$_m$ or $C_1$-$C_4$alkyl-[NH-$C_1$-$C_4$alkylene]$_m$, each of which is unsubstituted or substituted by one or more, where applicable identical or different, radicals $R_{17}$, or $C_6$-$C_{12}$aryl unsubstituted or substituted by one or more, where applicable identical or different, radicals $R_{18}$;

$R_{15}$ is hydrogen, cyano, COO-$R_{25}$ or $C_1$-$C_{12}$alkyl;

$R_{17}$ is halogen, hydroxy, O-$R_{25}$, cyano, COO-$R_{25}$ or carboxy; and

$R_{18}$ is halogen, nitro, cyano, O-$R_{19}$, CH=C$(CN)_2$, $COOR_{19}$, ureido, $CONR_{25}R_{26}$, $SO_2R_{25}$, P(=O)$OR_{19}OR_{23}$ or unsubstituted or substituted $C_1$-$C_{12}$alkyl.

**3.** An optical recording medium according to claim 1 or 2, wherein the recording layer comprises a compound of formula (I) wherein

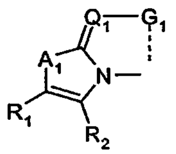

and/or

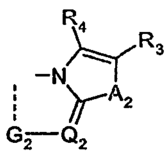 is/are 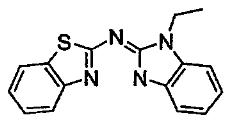, 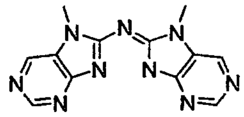,

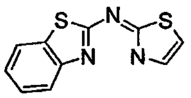, 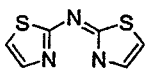, 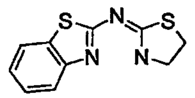, 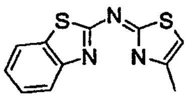,

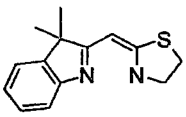, 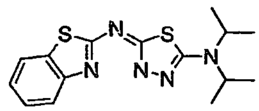, 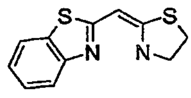, 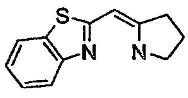,

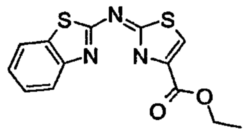 or 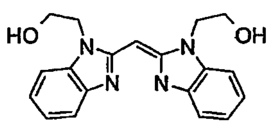.

4. An optical recording medium according to claim 1, 2 or 3, wherein the compound of formula (I) contains branched $C_3$-$C_{24}$alkyl or branched $C_3$-$C_{24}$alkenyl.

5. An optical recording medium according to claim 1, 2, 3 or 4, wherein the recording layer is substantially amorphous.

6. An optical recording medium according to claim 1, 2, 3, 4 or 5, additionally comprising a covering layer, wherein substrate, reflector layer, recording layer and covering layer are arranged in that order.

7. An optical recording medium according to claim 1, 2, 3, 4, 5 or 6, which in addition to comprising a compound of formula (I) comprises a metal-free chromophore.

8. A method of recording or playing back data, wherein the data on an optical recording medium according to claim 1, 2, 3, 4, 5, 6 or 7 are recorded or played back at a wavelength of from 350 to 500 nm.

9. A compound of formula (I) according to claim 1, with the proviso that the compound is not a compound of formula $M_2(Z_1)_2$, wherein:

- $M_2$ is Co(II), Cu(II), Hg(II), Ni(II), Pd(II) or Zn(II) and $Z_1$ is

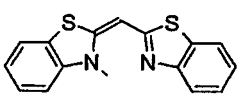;

- $M_2$ is Co(II), Cu(II), Ni(II), Pd(II) or Zn(II) and $Z_1$ is

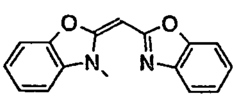,

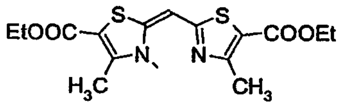 , 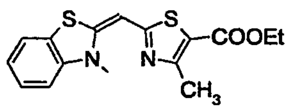 ,

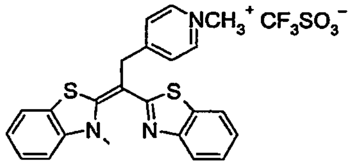 or 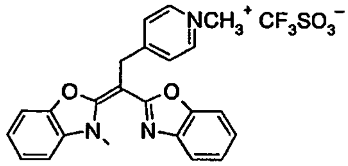 ;

• $M_2$ is Co(II), Cu(II), Ni(II) or Zn(II) and $Z_1$ is

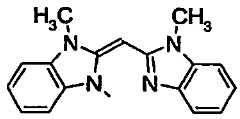 ;

• $M_2$ is Cu(II), Pd(II) or Zn(II) and $Z_1$ is

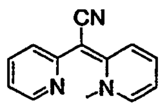 ;

• $M_2$ is Co(II), Cu(II) or Zn(II) and $Z_1$ is

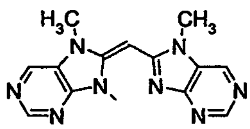 or 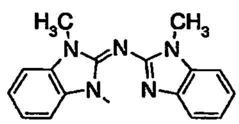 ;

• $M_2$ is Cu(II) or Zn(II) and $Z_1$ is

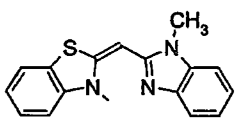 , 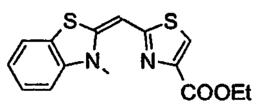 or 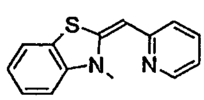 ;

• $M_2$ is Co(II) or Cu(II) and $Z_1$ is

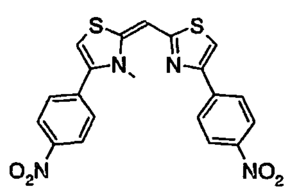 ;

- $M_2$ is Pd(II) or Zn(II) and $Z_1$ is

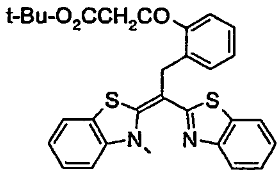 or 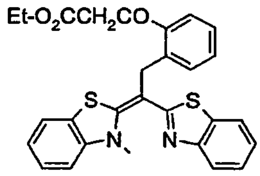 ;

- $M_2$ is Cu(II) and $Z_1$ is

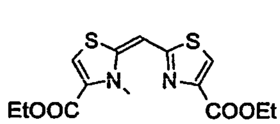 or 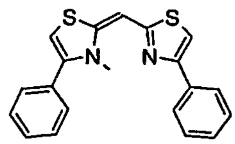 ;

or

- $M_2$ is Zn(II) and $Z_1$ is

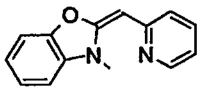 or 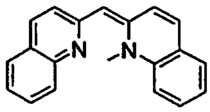 .

**10.** A compound according to claim 9, containing branched $C_3$-$C_{24}$alkyl or branched $C_3$-$C_{24}$alkenyl.

**11.** A compound according to claim 9 or 10 of formula (I), wherein

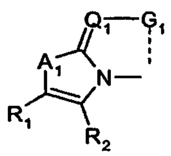

and/or

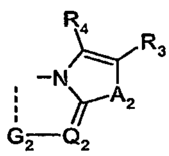 is/are 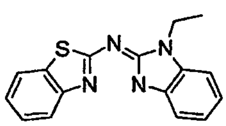 , 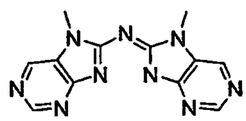 ,

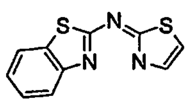 , 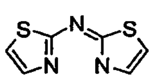 , 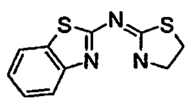 , 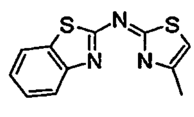 ,

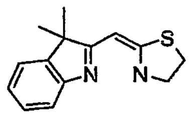, 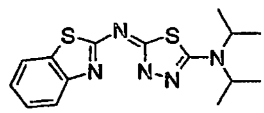, 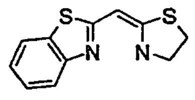, 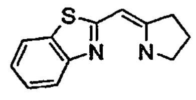,

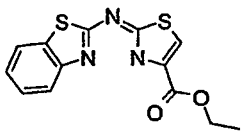 or 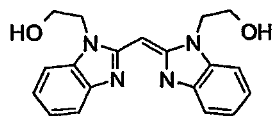.

## Patentansprüche

1. Optisches Aufzeichnungsmedium, enthaltend ein Substrat, eine Aufzeichnungsschicht und gegebenenfalls eine oder mehrere reflektierende Schichten, **dadurch gekennzeichnet, dass** die Aufzeichnungsschicht eine Verbindung der Formel

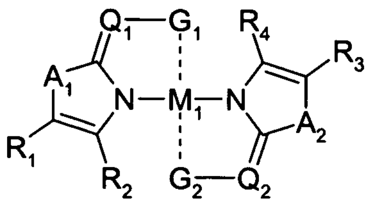 (I)

oder ein Tautomer davon enthält, worin
$G_1$ und $G_2$ unabhängig voneinander

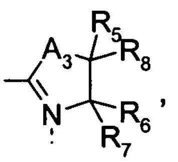, 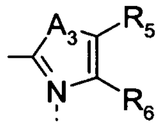, 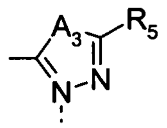 oder 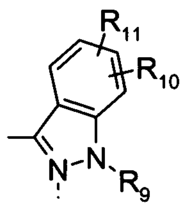

bedeuten,
$A_1$ und $A_2$ unabhängig voneinander für $N(R_{12})$, O, S oder Se und $A_3$ für $C(C_1\text{-}C_5Alkyl)_2$ , $C(C_4\text{-}C_5Alkylen)$, $N(R_{12})$, O, S, Se, $N{=}C(R_{13})$ oder unsubstituiertes oder mit $R_{14}$ substituiertes CH=CH stehen;
$M_1$ ein Übergangsmetall der Gruppen 9 bis 12 ist, bevorzugt Co, Cu, Ni, Pd oder Zn, besonders bevorzugt Co, Cu oder Ni,
$Q_1$ und $Q_2$ unabhängig voneinander für $C(R_{15})$, N oder P stehen,
$R_1$, $R_2$, $R_3$, $R_4$, $R_5$, $R_6$, $R_7$, $R_8$ und $R_{14}$ unabhängig voneinander für Wasserstoff, $R_{18}$ oder unsubstituiertes oder mit einem oder mehreren, gegebenenfalls identischen oder unterschiedlichen Resten $R_{18}$ substituiertes $C_6\text{-}C_{12}$Aryl, $C_4\text{-}C_{12}$Heteroaryl, $C_7\text{-}C_{12}$Aralkyl oder $C_5\text{-}C_{12}$Heteroaralkyl stehen; oder
$R_1$ und $R_2$, $R_3$ und $R_4$, $R_5$ und $R_6$, $R_5$ und $R_{13}$ und/oder $R_5$ und $R_{14}$ gemeinsam zu zweit unsubstituiertes oder mit einem oder mehreren, gegebenenfalls identischen oder unterschiedlichen Resten $R_{17}$ substituiertes $C_3\text{-}C_6$Alkylen oder $C_3\text{-}C_6$Alkenylen, welche gegebenenfalls durch O, S oder $N(R_{12})$ unterbrochen sein können, oder unsubstituiertes oder mit einem oder mehreren, gegebenenfalls identischen oder unterschiedlichen Resten $R_{18}$ substituiertes 1,4-Buta-1,3-dienylen,

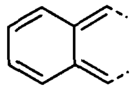

oder

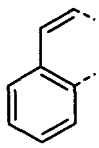

worin gegebenenfalls 1 oder 2 C durch N ersetzt sein können, bedeuten;

$R_9$, $R_{12}$ and $R_{13}$ unabhängig voneinander unsubstituiertes oder mit einem oder mehreren, gegebenenfalls identischen oder unterschiedlichen Resten $R_{17}$ substituiertes $C_1$-$C_{24}$Alkyl, $C_3$-$C_{24}$Cycloalkyl, $C_2$-$C_{24}$Alkenyl, $C_3$-$C_{24}$Cycloalkenyl, $C_1$-$C_4$Alkyl-[O-$C_1$-$C_4$alkylen]$_m$ oder $C_1$-$C_4$Alkyl-[NH-$C_1$-$C_4$alkylen]$_m$; oder unsubstituiertes oder mit einem oder mehreren, gegebenenfalls identischen oder unterschiedlichen Resten $R_{18}$ substituiertes $C_6$-$C_{12}$Aryl, $C_4$-$C_{12}$Heteroaryl, $C_7$-$C_{12}$Aralkyl oder $C_5$-$C_{12}$Heteroaralkyl stehen;

$R_{10}$, $R_{11}$ und $R_{18}$ unabhängig voneinander für Halogen, Nitro, Cyano, Thiocyanato, Hydroxy, O-$R_{19}$, O-CO-$R_{19}$, S-$R_{19}$, CHO, $COR_{20}$, $CHOR_{19}OR_{23}$ , $CR_{20}OR_{19}OR_{23}$, $R_{16}$, N=N-$R_{16}$, N=$CR_{19}R_{20}$, N=$CR_{21}R_{22}$, C($R_{15}$)=$NR_{19}$, C($R_{15}$)=$NR_{21}$, C($R_{15}$)=$CR_{21}R_{22}$, $NH_2$, NH-$R_{19}$, $NR_{19}R_{20}$, $NH_3^+$, $NH_2R_{19}^+$, $NHR_{19}R_{20}^+$, $NR_{19}R_{20}R_{23}^+$, $CONH_2$, $CONHR_{19}$, $CONR_{19}R_{20}$, $SO_2R_{19}$, $SO_2NH_2$, $SO_2NHR_{19}$, $SO_2NR_{19}R_{20}$, COOH, $COOR_{19}$, $OCOOR_{19}$, $NHCOR_{19}$, $NR_{19}COR_{23}$, $NHCOOR_{19}$, $NR_{19}COOR_{23}$, Ureido, $NR_{19}$-CO-$NHR_{23}$, $B(OH)_2$, B(OH)($OR_{19}$), B($OR_{19}$)$OR_{23}$, Phosphato, $PR_{19}R_{23}$, $POR_{19}OR_{23}$, P(=O)$OR_{19}OR_{23}$, $OPR_{19}R_{23}$, $OPR_{19}OR_{23}$, OP(=O)$R_{19}OR_{23}$, OP(=O)$OR_{19}OR_{23}$ , $OPO_3R_{19}$, Sulfato, Sulfo oder unsubstituiertes oder mit einem oder mehreren, gegebenenfalls identischen oder unterschiedlichen Resten $R_{17}$ substituiertes $C_1$-$C_{12}$Alkyl, $C_3$-$C_{12}$Cycloalkyl, $C_1$-$C_{12}$Alkylthio, $C_3$-$C_{12}$Cycloalkylthio, $C_1$-$C_{12}$Alkoxy oder $C_3$-$C_{12}$Cycloalkoxy steht;

$R_{15}$ für Wasserstoff, Cyano, Hydroxy, $C_1$-$C_{12}$Alkoxy, $C_3$-$C_{12}$Cycloalkoxy, $C_1$-$C_{12}$Alkylthio, $C_3$-$C_{12}$Cycloalkylthio, Amino, $NHR_{24}$, $NR_{25}R_{26}$, $R_{27}$, Halogen, Nitro, Formyl, N=N-$R_{27}$, C($R_{14}$)=$CR_{21}R_{22}$, C($R_{14}$)=$NR_{19}$, COO-$R_{25}$ , Carboxy, Carbamoyl, CONH-$R_{25}$ , $CONR_{25}R_{26}$ , N=$CR_{19}R_{20}$ oder unsubstituiertes oder mit einem oder mehreren, gegebenenfalls identischen oder unterschiedlichen Resten Halogen, Hydroxy, $C_1$-$C_{12}$Alkoxy oder $C_3$-$C_{12}$Cycloalkoxy substituiertes $C_1$-$C_{12}$Alkyl, $C_3$-$C_{12}$Cycloalkyl, $C_2$-$C_{12}$Alkenyl oder $C_3$-$C_{12}$Cycloalkenyl steht;

$R_{16}$ für unsubstituiertes oder mit einem oder mehreren, gegebenenfalls identischen oder unterschiedlichen Resten $R_{28}$ substituiertes $C_6$-$C_{12}$Aryl, $C_4$-$C_{12}$Heteroaryl, $C_7$-$C_{12}$Aralkyl oder $C_5$-$C_{12}$Heteroaralkyl steht;

$R_{17}$ für Halogen, Hydroxy, O-$R_{25}$ , O-CO-$R_{25}$ , S-$R_{25}$ , $NH_2$ , NH-$R_{25}$, $NR_{25}R_{26}$ , $NH_3^+$, $NH_2R_{25}^+$, $NHR_{25}R_{26}^+$, $NR_{24}R_{25}R_{26}^+$, $NR_{25}$-CO-$R_{24}$, $NR_{25}COOR_{24}$, Cyano, Formyl, COO-$R_{25}$, Carboxy, Carbamoyl, CONH-$R_{25}$, $CONR_{25}R_{26}$, Ureido, NH-CO-$NHR_{24}$, $NR_{25}$-CO-$NHR_{24}$ , Phosphato, $PR_{25}R_{24}$ , $POR_{25}OR_{24}$ , P(=O)$OR_{25}OR_{24}$ , $OPR_{25}R_{24}$ , $OPR_{25}OR_{24}$ , OP(=O)$R_{25}OR_{24}$ , $OPO_3R_{25}$ , OP(=O)$OR_{25}OR_{24}$ , $SO_2R_{25}$ , Sulfato, Sulfo, $R_{27}$, N=N-$R_{27}$ oder unsubstituiertes oder einfach oder mehrfach mit Halogen substituiertes $C_1$-$C_{12}$Alkaxy oder $C_1$-$C_{12}$Cycloalkoxy steht;

$R_{19}$, $R_{20}$ und $R_{23}$ unabhängig voneinander für $R_{16}$ oder unsubstituiertes oder mit einem oder mehreren, gegebenenfalls identischen oder unterschiedlichen Resten Halogen, Hydroxy, $C_1$-$C_{12}$Alkoxy oder $C_3$-$C_{12}$Cycloalkaxy substituiertes $C_1$-$C_{12}$Alkyl, $C_3$-$C_{12}$Cycloalkyl, $C_2$-$C_{12}$Alkenyl oder $C_3$-$C_{12}$Cycloalkenyl stehen; oder

$R_{14}$ und $R_{19}$ , $R_{15}$ und $R_{19}$ und/oder $R_{19}$ und $R_{23}$ zusammen für unsubstituiertes oder mit einem oder mehreren, gegebenenfalls identischen oder unterschiedlichen Resten Halogen, Hydroxy, $C_1$-$C_{12}$Alkoxy oder $C_3$-$C_{12}$Cycloalkoxy substituiertes $C_2$-$C_{12}$Alkylen, $C_3$-$C_{12}$Cycloalkylen, $C_2$-$C_{12}$Alkenylen oder $C_3$-$C_{12}$Cycloalkenylen stehen; oder

$R_{19}$ und $R_{20}$ zusammen mit dem gemeinsam N für unsubstituiertes oder mit $C_1$-$C_4$Alkyl einfach bis vierfach substituiertes Pyrrolidin, Piperidin, Piperazin oder Morpholin; oder unsubstituiertes oder mit einem oder mehreren, gegebenenfalls identischen oder unterschiedlichen Resten $R_{28}$ substituiertes Carbazol, Phenoxazin oder Phenothiazin stehen;

$R_{21}$ und $R_{22}$ unabhängig voneinander für $NR_{25}R_{26}$, CN, $CONH_2$, $CONHR_{19}$, $CONR_{19}R_{20}$ oder $COOR_{20}$ stehen;

$R_{24}$, $R_{25}$ und $R_{26}$ unabhängig voneinander für $C_1$-$C_{12}$Alkyl, $C_3$-$C_{12}$Cycloalkyl, $C_2$-$C_{12}$Alkenyl, $C_3$-$C_{12}$Cycloalkenyl, $C_6$-$C_{12}$Aryl, $C_4$-$C_{12}$Heteroaryl, $C_7$-$C_{12}$Aralkyl oder $C_5$-$C_{12}$Heteroaralkyl stehen; oder

$R_{25}$ und $R_{26}$ zusammen mit dem gemeinsam N für unsubstituiertes oder mit $C_1$-$C_4$Alkyl einfach bis vierfach substi-

tuiertes Pyrrolidin, Piperidin, Piperazin oder Morpholin stehen; $R_{27}$ für unsubstituiertes oder mit einem oder mehreren, gegebenenfalls identischen oder unterschiedlichen Resten $R_{18}$ substituiertes $C_6$-$C_{12}$Aryl, $C_4$-$C_{12}$Heteroaryl, $C_7$-$C_{12}$Aralkyl oder $C_5$-$C_{12}$Heteroaralkyl steht; $R_{28}$ für Nitro, $SO_2NHR_{25}$, $SO_2NR_{25}R_{26}$ oder unsubstituiertes oder mit einem oder mehreren, gegebenenfalls identischen oder unterschiedlichen Resten $R_{17}$ substituiertes $C_1$-$C_{12}$Alkyl, $C_3$-$C_{12}$Cycloalkyl, $C_1$-$C_{12}$Alkylthio, $C_3$-$C_{12}$Cycloalkylthio, $C_1$-$C_{12}$Alkoxy oder $C_3$-$C_{12}$Cycloalkoxy; und m für eine Zahl von 1 bis 10 steht.

**2.** Optisches Aufzeichnungsmedium gemäss Anspruch 1, worin $Q_1$ und $Q_2$ für $C(R_{15})$; $G_1$ und $G_2$ für

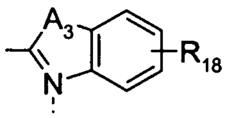

und $A_1$, $A_2$ und $A_3$ für O, S oder $N(R_{12})$ stehen; $R_{12}$ für unsubstituiertes oder mit einem oder mehreren, gegebenenfalls identischen oder unterschiedlichen Resten $R_{17}$ substituiertes $C_1$-$C_{24}$Alkyl, $C_1$-$C_4$Alkyl-[O-$C_1$-$C_4$alkylen]$_m$ oder $C_1$-$C_4$Alkyl-[NH-$C_1$-$C_4$alkylen]$_m$, oder unsubstituiertes oder mit einem oder mehreren, gegebenenfalls identischen oder unterschiedlichen Resten $R_{18}$ substituiertes $C_6$-$C_{12}$Aryl steht; $R_{15}$ für Wasserstoff, Cyano, COO-$R_{25}$ oder $C_1$-$C_{12}$Alkyl steht; $R_{17}$ für Halogen, Hydroxy, O-$R_{25}$, Cyano, COO-$R_{25}$ oder Carboxy steht; und $R_{18}$ für Halogen, Nitro, Cyano, O-$R_{19}$, $CH=C(CN)_2$, $COOR_{19}$, Ureido, $CONR_{25}R_{26}$, $SO_2R_{25}$, $P(=O)OR_{19}OR_{23}$ oder unsubstituiertes oder substituiertes $C_1$-$C_{12}$Alkyl steht.

**3.** Optisches Aufzeichnungsmedium gemäss Anspruch 1 oder 2, worin die Aufzeichnungsschicht eine Verbindung der Formel (I) enthält, worin

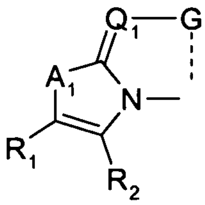 und/oder 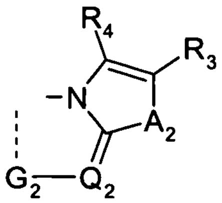 für 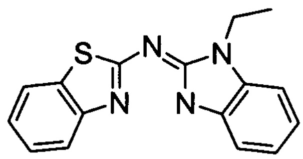 ,

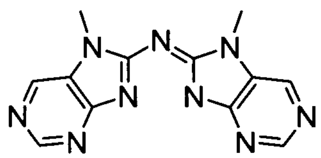 , 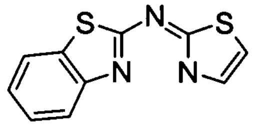 , 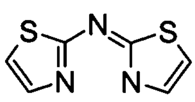 , 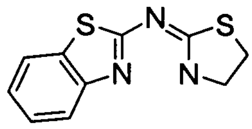 ,

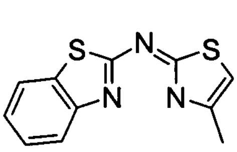 , 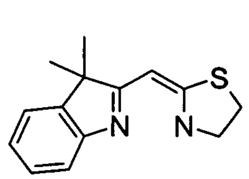 , 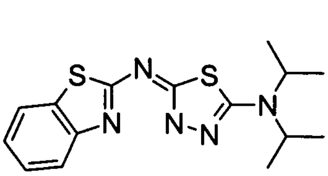 , 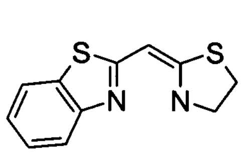 ,

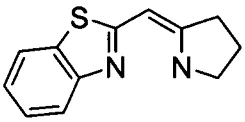 , 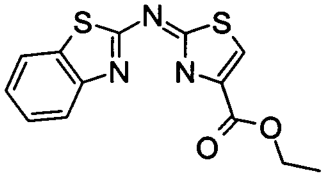 oder 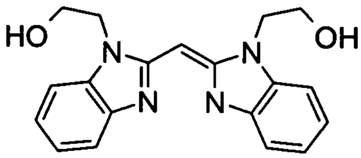

stehen.

4. Optisches Aufzeichnungsmedium gemäss Anspruch 1, 2 oder 3, worin die Verbindung der Formel (I) verzweigtes $C_3$-$C_{24}$Alkyl oder verzweigtes $C_3$-$C_{24}$Alkenyl enthält.

5. Optisches Aufzeichnungsmedium gemäss Anspruch 1, 2, 3 oder 4, worin die Aufzeichnungsschicht im wesentlichen amorph ist.

6. Optisches Aufzeichnungsmedium gemäss Anspruch 1, 2, 3, 4 oder 5, enthaltend zusätzlich eine Deckschicht, wobei Substrat, Reflektorschicht, Aufzeichnungsschicht und Deckschicht in dieser Reihenfolge angeordnet sind.

7. Optisches Aufzeichnungsmedium gemäss Anspruch 1, 2, 3, 4, 5 oder 6, welches zusätzlich zur Verbindung der Formel (I) ein metallfreies Chromophor enthält.

8. Verfahren zur Aufzeichnung oder Wiedergabe von Daten, **dadurch gekennzeichnet, dass** die Daten auf einem optischen Aufzeichnungsmedium gemäss Anspruch 1, 2, 3, 4, 5, 6 oder 7, bei einer Wellenlänge von 350 bis 500 nm aufgezeichnet oder wiedergegeben werden.

9. Verbindung der Formel (I) gemäss Anspruch 1, mit der Massgabe, dass die Verbindung nicht der Formel $M_2(Z_1)_2$ ist, worin:

- $M_2$ Co(II), Cu(II), Hg(II), Ni(II), Pd(II) oder Zn(II) und $Z_1$

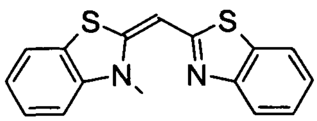 ;

- $M_2$ Co(II), Cu(II), Ni(II), Pd(II) oder Zn(II) und $Z_1$

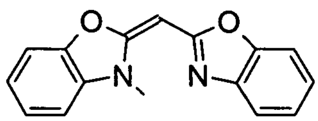 ,

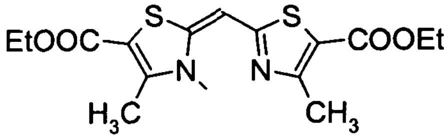 , 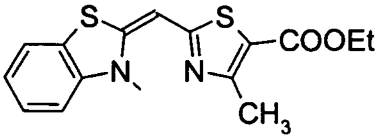 ,

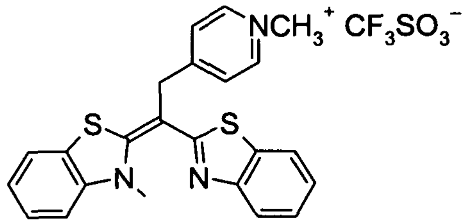 oder 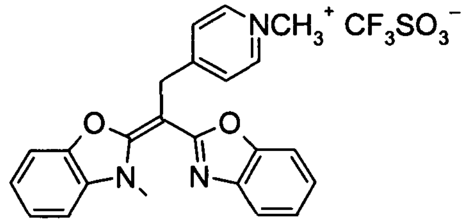 ;

• $M_2$ Co(II), Cu(II), Ni(II) oder Zn(II) und $Z_1$

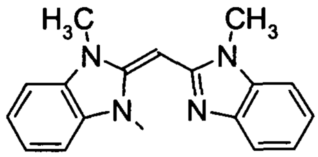 ;

• $M_2$ Cu(II), Pd(II) oder Zn(II) und $Z_1$

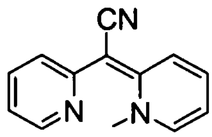 ;

• $M_2$ Co(II), Cu(II) oder Zn(II) und $Z_1$

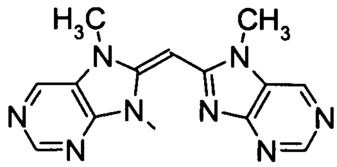 oder 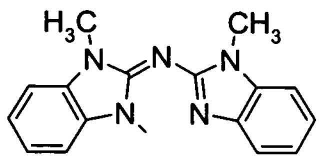 ;

• $M_2$ Cu(II) oder Zn(II) und $Z_1$

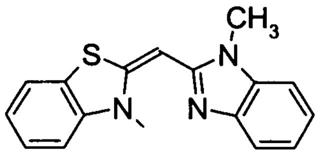 , 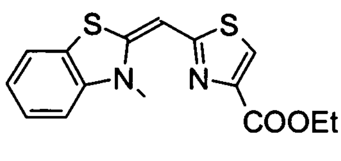 oder 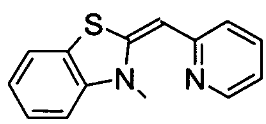 ;

• $M_2$Co(II) oder Cu(II) und $Z_1$

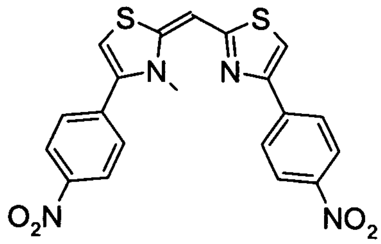

;

• $M_2$ Pd(II) oder Zn(II) und $Z_1$

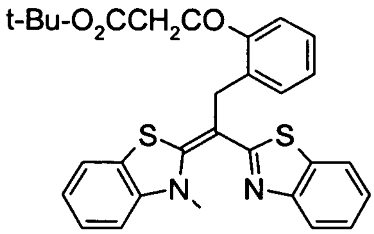

oder

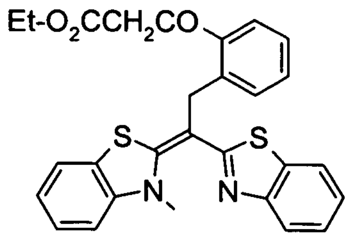

;

• $M_2$ Cu(II) und $Z_1$

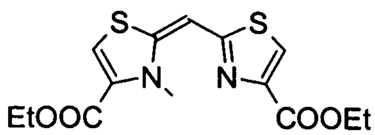

oder

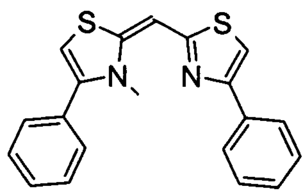

;

oder

• $M_2$ Zn(II) und $Z_1$

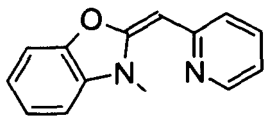

oder

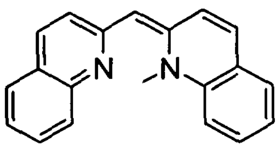

bedeuten.

**10.** Verbindung gemäss Anspruch 9, welche verzweigtes $C_3$-$C_{24}$Alkyl oder verzweigtes $C_3$-$C_{24}$Alkenyl enthält.

**11.** Verbindung gemäss Anspruch 9 oder 10 der Formel (I), worin

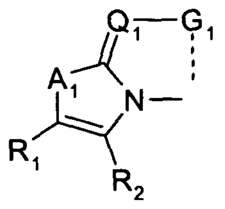

und/oder

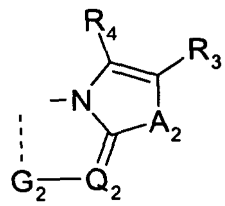 für 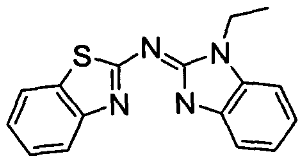, 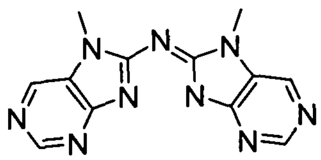,

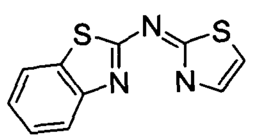, 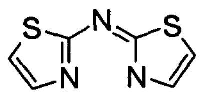, 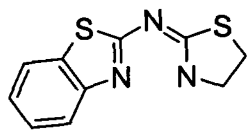, 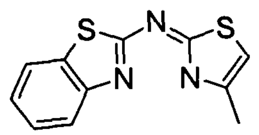,

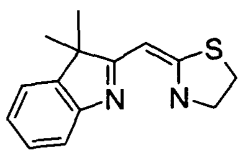, 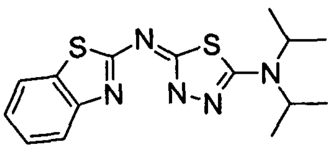, 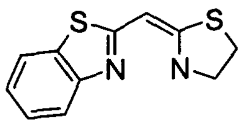, 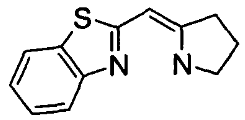,

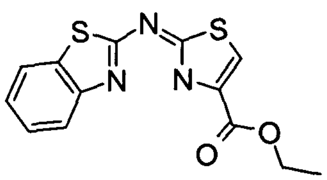 oder 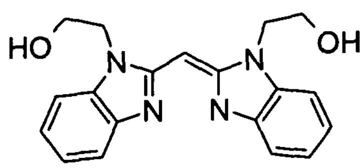

stehen.

## Revendications

**1.** Milieu d'enregistrement optique comprenant un substrat, une couche d'enregistrement et éventuellement une ou plusieurs couches réfléchissantes, dans lequel la couché d'enregistrement comprend un composé de formule

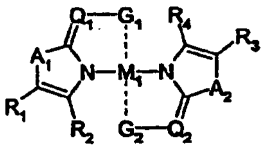 (I)

ou un de ses tautomères, dans lequel $G_1$ et $G_2$ représentent chacun, indépendamment l'un de l'autre,

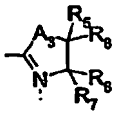, 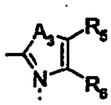, 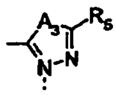 ou 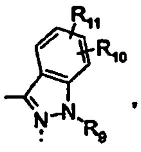,

$A_1$ et $A_2$ représentent chacun, indépendamment l'un de l'autre, $N(R_{12})$, O, S ou Se et $A_3$ représente C (alkyle en $C_1$ à $C_5$)$_2$, C(alkylène en $C_4$ à $C_5$), $N(R_{12})$, O, S, Se, $N{=}C(R_{13})$ ou CH=CH non substitué ou à substitution $R_{14}$ ;
$M_1$ représente un métal de transition des groupes IX à XII, de préférence Co, Cu, Ni, Pd ou Zn, en particulier Co, Cu ou Ni ;
$Q_1$ et $Q_2$ représentent chacun, indépendamment l'un de l'autre, $C(R_{15})$, N ou P ;
$R_1$, $R_2$, $R_3$, $R_4$, $R_5$, $R_6$, $R_7$, $R_8$ et $R_{14}$ représentent chacun, indépendamment les uns des autres, un hydrogène, $R_{18}$, ou un groupe aryle en $C_6$ à $C_{12}$, hétéroaryle en $C_4$ à $C_{12}$, aralkyle en $C_7$ à $C_{12}$ ou hétéroaralkyle en $C_5$ à $C_{12}$ chacun non substitué ou substitué par un ou plusieurs radicaux $R_{18}$, le cas échéant identiques ou différents ; ou $R_1$ et $R_2$, $R_3$ et $R_4$, $R_5$ et $R_6$, $R_5$ et $R_{13}$ et/ou $R_5$ et $R_{14}$, ensemble par paire, représentent un groupe alkylène en $C_3$ à $C_6$ ou alcénylène en $C_3$ à $C_6$, dont chacun est non substitué ou substitué par un ou plusieurs radicaux $R_{17}$, le cas échéant identiques ou différents, et peuvent être ininterrompus ou interrompus par 0, S ou $N(R_{12})$, ou 1,4-buta-1,3-diénylène,

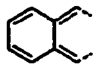

ou

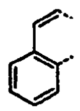.

dont chacun est non substitué ou substitué par un ou plusieurs radicaux $R_{18}$, le cas échéant identiques ou différents, et dans lesquels 1 ou 2 atomes de carbone peuvent avoir été remplacés par un azote ;
Rg, $R_{12}$ et $R_{13}$ représentent chacun, indépendamment les uns des autres, un groupe alkyle en $C_1$ à $C_{24}$, cycloalkyle en $C_3$ à $C_{24}$, alcényle en $C_2$ à $C_{24}$, cycloalcényle en $C_3$ à $C_{24}$, (alkyle en $C_1$ à $C_4$)-[O-(alkylène en $C_1$ à $C_4$)]$_m$ ou (alkyle en $C_1$ à $C_4$)-[NH-(alkylène en $C_1$ à $C_4$)]$_m$, dont chacun est non substitué ou substitué par un ou plusieurs radicaux $R_{17}$, le cas échéant identiques ou différents ; ou aryle en $C_6$ à $C_{12}$, hétéroaryle en $C_4$ à $C_{12}$, aralkyle en $C_7$ à $C_{12}$ ou hétéroaralkyle en $C_5$ à $C_{12}$, dont chacun est non substitué ou substitué par un ou plusieurs radicaux $R_{18}$, le cas échéant identiques ou différents ; $R_{10}$, $R_{11}$ et $R_{15}$ représentent chacun, indépendamment les uns des autres, un halogène, un groupe nitro, cyano, thiocyanato, hydroxyle, $O{-}R_{19}$, $O{-}CO{-}R_{19}$, $S{-}R_{19}$, CHO, $COR_{20}$, $CHOR_{19}OR_{23}$, $CR_{20}OR_{19}OR_{23}$, $R_{16}$, $N{=}N{-}R_{16}$, $N{=}C\ R_{19}R_{20}$, $N{=}CR_{21}R_{22}$, C $(R_{15})$ $=NR_{19}$, $C(R_{15})$ $=NR_{21}$ $C(R_{15})$ $=CR_{21}R_{22}$, $NH_2$, $NH{-}R_{19}$, $NR_{19}R_{20}$, $NH_3^+$, $NH_2R_{19}^+$, $NHR_{19}R_{20}^+$, $NR_{19}R_{20}R_{23}^+$, $CONH_2$, $CONHR_{19}$, $CONR_{19}R_{20}$, $SO_2R_{19}$, $SO_2NH_2$, $SO_2NHR_{19}$, $SO_2NR_{19}R_{20}$, COOH, $COOR_{19}$, $OCOOR_{19}$, $NHCOR_{19}$, $NR_{19}COR_{23}$, $NHCOOR_{19}$, $NR_{19}COOR_{23}$, uréido, $NR_{19}{-}CO{-}NHR_{23}$, B $(OH)_2$, B(OH) $(OR_{19})$, $B(OR_{19})OR_{23}$, phosphato, $PR_{19}R_{23}$, $POR_{19}OR_{23}$, $P(=O)OR_{19}OR_{23}$, $OPR_{19}R_{23}$, $OPR_{19}OR_{23}$, OP(=O) $R_{19}OR_{23}$, OP(=O) $OR_{19}OR_{23}$, $OPO_3R_{19}$, sulfato, sulfo, ou alkyle

en $C_1$ à $C_{12}$, cycloalkyle en $C_3$ à $C_{12}$, alkylthio en $C_1$ à $C_{12}$, cycloalkylthio en $C_3$ à $C_{12}$, alcoxy en $C_1$ à $C_{12}$ ou cycloalcoxy en $C_3$ à $C_{12}$, chacun non substitué ou substitué par un ou plusieurs radicaux $R_{17}$, le cas échéant identiques ou différents ;

$R_{15}$ représente un hydrogène, un groupe cyano, hydroxyle, alcoxy en $C_1$ à $C_{12}$, cycloalcoxy en $C_3$ à $C_{12}$, alkylthio en $C_1$ à $C_{12}$, cycloalkylthio en $C_3$ à $C_{12}$, amino, $NHR_{24}$, $NR_{25}R_{26}$, $R_{27}$, halogéno, nitro, formyle, N=N-$R_{27}$, $C(R_{14})=CR_{21}R_{22}$, $C(R_{14})=NR_{19}$, COO-$R_{25}$, carboxyle, carbamoyle, CONH-$R_{25}$, $CONR_{25}R_{26}$, $N=CR_{19}R_{20}$, ou alkyle en $C_1$ à $C_{12}$, cycloalkyle en $C_3$ à $C_{12}$, alcényle en $C_2$ à $C_{12}$ ou cycloalcényle en $C_3$ à $C_{12}$, chacun non substitué ou substitué par un ou plusieurs radicaux halogéno, hydroxyle, alcoxy en $C_1$ à $C_{12}$ ou cycloalcoxy en $C_3$ à $C_{12}$, le cas échéant identiques ou différents ;

$R_{16}$ représente un groupe aryle en $C_6$ à $C_{12}$, hétéroaryle en $C_4$ à $C_{12}$, aralkyle en $C_7$ à $C_{12}$ ou hétéroaralkyle en $C_5$ à $C_{12}$, dont chacun est non substitué ou substitué par un ou plusieurs radicaux $R_{28}$, le cas échéant identiques ou différents ;

$R_{17}$ représente un atome d'halogène, un groupe hydroxyle, O-$R_{25}$, O-CO-$R_{25}$, S-$R_{25}$, $NH_2$, NH-$R_{25}$, $NR_{25}R_{26}$, $NH_3^+$, $NH_2R_{25}^+$, $NHR_{25}R_{26}^+$, $NR_{24}R_{25}R_{26}^+$, $NR_{25}$-CO-$R_{24}$, $NR_{25}COOR_{24}$, cyano, formyle, COO-$R_{25}$, carboxyle, carbamoyle, CONH-$R_{25}$, $CONR_{25}R_{26}$, uréido, NH-CO-$NHR_{24}$, $NR_{25}$-CO-$NHR_{24}$, phosphato, $PR_{25}R_{24}$ $POR_{25}OR_{24}$, $P(=O)OR_{25}OR_{24}$, $OPR_{25}R_{24}$, $OPR_{25}OR_{24}$, $OP(=O)R_{25}OR_{24}$, $OPO_3R_{25}$, $OP(=O)OR_{25}OR_{24}$, $SO_2R_{25}$, sulfato, sulfo, $R_{27}$, N=N-$R_{27}$, ou alcoxy en $C_1$ à $C_{12}$ ou cycloalcoxy en $C_1$ à $C_{12}$, chacun non substitué ou mono- ou poly-substitué par un atome d'halogène ;

$R_{19}$, $R_{20}$ et $R_{23}$ représentent chacun, indépendamment les uns des autres, $R_{16}$, ou un groupe alkyle en $C_1$ à $C_{12}$, cycloalkyle en $C_3$ à $C_{12}$, alcényle en $C_2$ à $C_{12}$ ou cycloalcényle en $C_3$ à $C_{12}$, dont chacun est non substitué ou substitué par un ou plusieurs radicaux halogéno, hydroxyle, alcoxy en $C_1$ à $C_{12}$ ou cycloalcoxy en $C_3$ à $C_{12}$ le cas échéant identiques ou différents ; ou

$R_{14}$ et $R_{19}$ ensemble, $R_{15}$ et $R_{19}$ ensemble et/ou $R_{19}$ et $_{23}$ ensemble représentent un groupe alkylène en $C_2$ à $C_{12}$, cycloalkylène en $C_3$ à $C_{12}$, alcénylène en $C_2$ à $C_{12}$ ou cycloalcénylène en $C_3$ à $C_{12}$, dont chacun est non substitué ou substitué par un ou plusieurs radicaux halogéno, hydroxyle, alcoxy en $C_1$ à $C_{12}$ ou cycloalcoxy en $C_3$ à $C_{12}$, le cas échéant identiques ou différents ; ou

$R_{19}$ et $R_{20}$ conjointement avec l'atome d'azote commun représentent la pyrrolidine, la pipéridine, la pipérazine ou la morpholine, dont chacun est non substitué ou mono- à tétra-substitué par un groupe alkyle en $C_1$ à $C_4$; ou le carbazole, la phénoxazine ou la phénothiazine, dont chacun est non substitué ou substitué par un ou plusieurs radicaux $R_{28}$, le cas échéant identiques ou différents, ; $R_{21}$ et $R_{22}$ représentent chacun, indépendamment l'un de l'autre, $NR_{25}R_{26}$, CN, $CONH_2$, $CONHR_{19}$, $CONR_{19}R_{20}$ ou $COOR_{20}$;

$R_{24}$, $R_{25}$ et $R_{26}$ représentent chacun, indépendamment les uns des autres, un groupe alkyle en $C_1$ à $C_{12}$, cycloalkyle en $C_3$ à $C_{12}$, alcényle en $C_2$ à $C_{12}$, cycloalcényle en $C_3$ à $C_{12}$, aryle en $C_6$ à $C_{12}$, hétéroaryle en $C_4$ à $C_{12}$, aralkyle en $C_7$ à $C_{12}$ ou hétéroaralkyle en $C_5$ à $C_{12}$ ; ou

$R_{25}$ et $R_{26}$ conjointement avec l'atome d'azote commun représentent la pyrrolidine, la pipéridine, la pipérazine ou la morpholine, dont chacun est non substitué ou mono- à tétra-substitué par un groupe alkyle en $C_1$ à $C_4$ ;

$R_{27}$ représente un groupe aryle en $C_6$ à $C_{12}$, hétéroaryle en $C_4$ à $C_{12}$, aralkyle en $C_7$ à $C_{12}$ ou hétéroaralkyle en $C_5$ à $C_{12}$, dont chacun est non substitué ou substitué par un ou plusieurs radicaux $R_{16}$, le cas échéant identiques ou différents ;

$R_{28}$ représente un groupe nitro, $SO_2NHR_{25}$, $SO_2NR_{25}R_{26}$, ou alkyle en $C_1$ à $C_{12}$, cycloalkyle en $C_3$ à $C_{12}$, alkylthio en $C_1$ à $C_{12}$, cycloalkylthio en $C_3$ à $C_{12}$, alcoxy en $C_1$ à $C_{12}$ ou cycloalcoxy en $C_3$ à $C_{12}$, dont chacun est non substitué ou substitué par un ou plusieurs radicaux $R_{17}$, le cas échéant identiques ou différents ; et

m représente un nombre valant 1 à 10.

**2.** Milieu d'enregistrement optique selon la revendication 1, dans lequel $Q_1$ et $Q_2$ représentent $C(R_{15})$;
$G_1$ et $G_2$ représentent

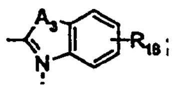

et $A_1$, $A_2$ et $A_3$ représentent 0, S ou $N(R_{12})$;

$R_{12}$ représente un groupe alkyle en $C_1$ à $C_{24}$, (alkyle en $C_1$ à $C_4$)-[O-(alkylène en $C_1$ à $C_4$)]$_m$ ou (alkyle en $C_1$ à $C_4$)-[NH-(alkylène en $C_1$ à $C_4$)]$_m$, dont chacun est non substitué ou substitué par un ou plusieurs radicaux $R_{17}$, le cas échéant identiques ou différents, ou aryle en $C_6$ à $C_{12}$ non substitué ou substitué par un ou plusieurs radicaux $R_{18}$, le cas échéant identiques ou différents ;

$R_{15}$ représente un atome d'hydrogène, un groupe cyano, COO-$R_{25}$ ou un groupe alkyle en $C_1$ à $C_{12}$ ;

$R_{17}$ représente un atome d'halogène, un groupe hydroxyle, O-$R_{25}$, cyano, COO-$R_{25}$ ou carboxyle ; et
$R_{18}$ représente un atome d'halogène, un groupe nitro, cyano, O-$R_{19}$ $CH=C(CN)_2$, $COOR_{19}$, uréido, $CONR_{25}R_{26}$, $SO_2R_{25}$, $P(=O)OR_{19}OR_{23}$ ou un groupe alkyle en $C_1$ à $C_{12}$ non substitué ou substitué.

**3.** Milieu d'enregistrement optique selon la revendication 1 ou 2, dans lequel la couche d'enregistrement comprend un composé de formule (I) dans laquelle

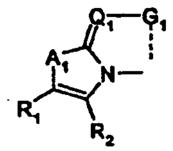

et/ou

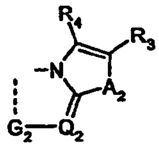 est/sont 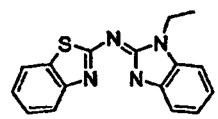, 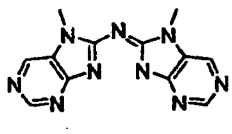,

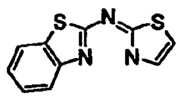, 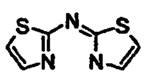, 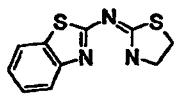, 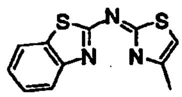,

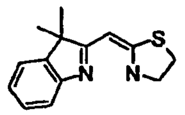, 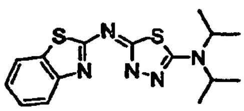, 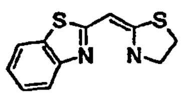, 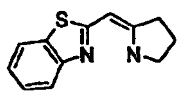,

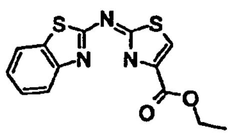 ou 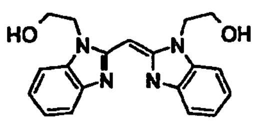.

**4.** Milieu d'enregistrement optique selon la revendication 1, 2 ou 3, dans lequel le composé de formule (I) contient un groupe alkyle en $C_3$ à $C_{24}$ ramifié ou alcényle en $C_3$ à $C_{24}$ ramifié.

**5.** Milieu d'enregistrement optique selon la revendication 1, 2, 3 ou 4, dans lequel la couche d'enregistrement est essentiellement amorphe.

**6.** Milieu d'enregistrement optique selon la revendication 1, 2, 3, 4 ou 5, comprenant en outre une couche de recouvrement, dans laquelle un substrat, une couche réfléchissante, une couche d'enregistrement et une couche de recouvrement sont disposées dans cet ordre.

**7.** Milieu d'enregistrement optique selon la revendication 1, 2, 3, 4, 5 ou 6, qui en plus de comprendre un composé de formule (I) comprend un chromophore exempt de métal.

**8.** Procédé d'enregistrement ou de lecture de données, dans lequel les données sur un milieu d'enregistrement optique selon la revendication 1, 2, 3, 4, 5, 6 ou 7 sont enregistrées ou lues à une longueur d'onde de 350 à 500 nm.

**9.** Composé de formule (I) selon la revendication 1, à condition que le composé ne soit pas un composé de formule

$M_2(Z_1)_2$, dans laquelle :

- $M_2$ représente Co(II), Cu(II), Hg(II), Ni(II), PD(II) ou Zn(II) et $Z_1$ représente

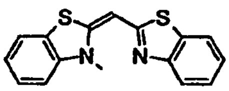 ;

- $M_2$ représente Co(II), Cu(II), Ni(II), Pd(II) ou Zn(II) et $Z_1$ représente

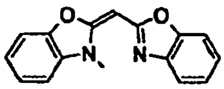 ,

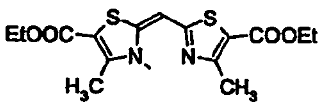 , 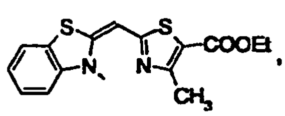 ,

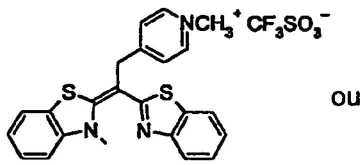 ou 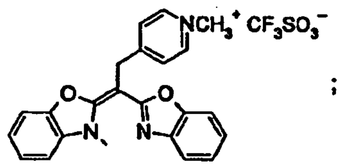 ;

- $M_2$ représente Co(II), Cu(II), Ni(II) ou Zn (II) et $Z_1$ représente

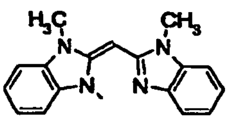 ;

- $M_2$ représente Cu(II), Pd(II) ou Zn(II) et $Z_1$ représente

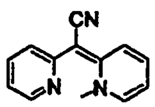 ;

- $M_2$ représente Co(II), Cu(II) ou Zn(II) et $Z_1$ représente

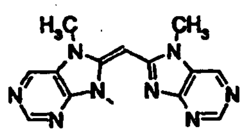 ou 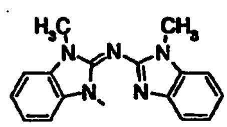 ;

- $M_2$ représente Cu(II) ou Zn(II) et $Z_1$ représente

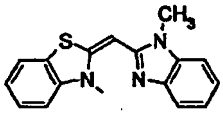 , 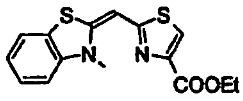 ou 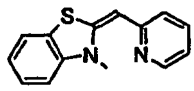 ;

- $M_2$ représente Co(II) ou Zn(II) et $Z_1$ représente

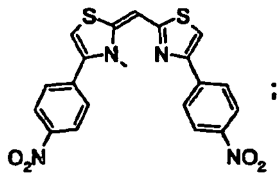 ;

- $M_2$ représente Pd(II) ou Zn(II) et $Z_1$ représente

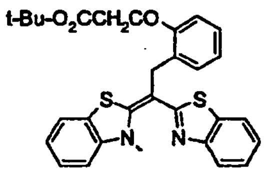 ou 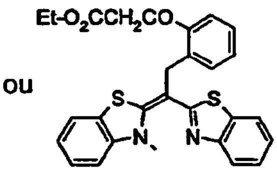 ;

- $M_2$ représente Cu(II) et $Z_1$ représente

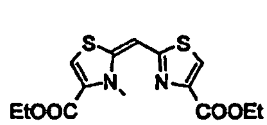 ou 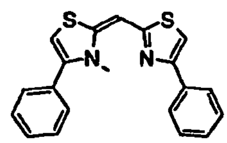 ;

ou

- $M_2$ représente Zn(II) et $Z_1$ représente

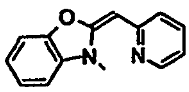 ou 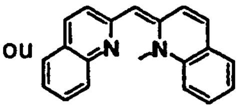 .

**10.** Composé selon la revendication 9 contenant un groupe alkyle en $C_3$ à $C_{24}$ ramifié ou alcényle en $C_3$ à $C_{24}$ ramifié.

**11.** Composé selon la revendication 9 ou 10 de formule (I), dans laquelle

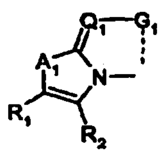

et/ou

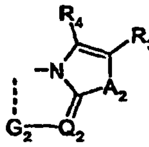 est/sont 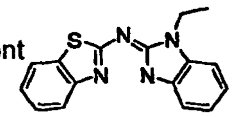, 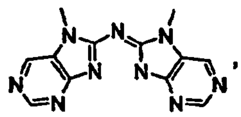,

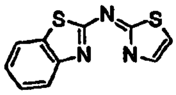, 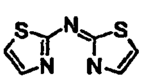, 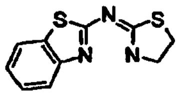, 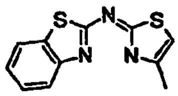,

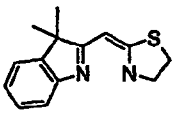, 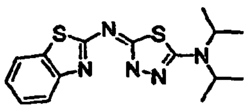, 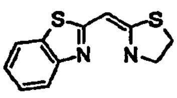, 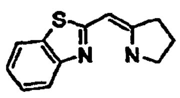,

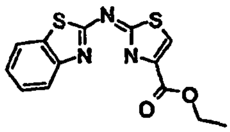 ou 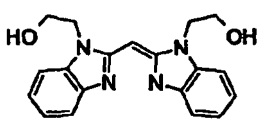.